# EUROPEAN PATENT SPECIFICATION

(11) **EP 3 068 542 B1**
(45) Date of publication and mention of the grant of the patent: **09.08.2023**
(21) Application number: 14806514.7
(22) Date of filing: 14.11.2014
(51) Int. Cl.: G01N 21/31, A61M 1/02, A61M 1/36, G01N 33/49, B04B 5/04, B04B 13/00

(54) **METHOD AND APPARATUS FOR CONTROLLING FLOW OF COMPONENTS SEPARATED FROM WHOLE BLOOD**
VERFAHREN UND VORRICHTUNG ZUM STEUERN DES FLUSSES VON AUS VOLLBLUT GETRENNTEN KOMPONENTEN
MÉTHODE ET APPAREIL POUR CONTRÔLER LE FLUX DE COMPOSANTS SÉPARÉS DU SANG TOTAL

(30) Priority: 15.11.2013 US 201361904715 P
(43) Date of publication of application: 21.09.2016
(73) Proprietor: Terumo BCT, Inc., Lakewood, CO 80215 (US)
(72) Inventor: JAMES, Craig, Arvada, Colorado 80004 (US); HUDOCK, Darryl, Centennial, Colorado 80122 (US); LEHMAN, John T., Corbett, Oregon 97019 (US)
(74) Representative: J A Kemp LLP
(86) International application number: PCT/US2014/065795
(87) International publication number: WO 2015/073886

(56) References cited:
- EP-A2- 1 512 464
- WO-A1-2008/105972
- US-A- 4 350 441
- US-A- 5 734 464
- US-A1- 2006 205 581
- US-A1- 2011 201 487

## Description

There are a number of processes that separate a composite fluid into its components. Some examples include biological fluids comprising an aqueous component and one or more cellular components, such as whole blood. Whole blood may be processed by extracting a plasma component and a cellular component (including platelets, white blood cells, and red blood cells) from a volume of whole blood. After separation, the components, such as plasma, platelets, white blood cells, and red blood cells may be collected separately.

WO 2008/105972 discloses a blood separation apparatus but does not teach using visible light to identify components. EP 1,512,464 describes a control device for use in collection of blood components that uses visible and infrared light to identify plasma, platelets, and red blood cells.

In order to facilitate the collection of different components of a composite fluid, once separated, there should be a way to identify the interface between a first component of the composite fluid and a second component of the composite fluid. For example, in the case of whole blood, it is desirable to identify the interface between the various components of whole blood after separation. Being able to identify the interface between two components, allows the component's to be removed from a container, in which the whole blood is separated, separately with minimal contamination between components. The ability to identify an interface between components can therefore be useful in obtaining relatively pure volumes of components in a composite fluid.

Embodiments of the present invention have been made in light of these and other considerations. However, the relatively specific problems discussed above do not limit the applicability of the embodiments of the present invention.

Embodiments are directed to the method for controlling a flow of components separated from whole blood according to claim 1.

Some embodiments provide for controlling the flow of components of whole blood after the components have been separated. For example, embodiments may involve separating a fluid comprising whole blood into components. A first valve is opened to allow a flow of plasma into a first collection bag. A first beam of a first light is emitted at the flow of plasma. A first photosensor then detects a first amount of the first light transmitted through the flow of plasma. A first beam of a second light is then emitted at the flow of plasma, and a second photosensor, detects a first amount of the second light transmitted through the flow of plasma. A baseline ratio of the first amount of the first light to the first amount of the second light is determined.

After a period of time, a second beam of the first light is emitted at the flow of plasma, and the first photosensor, detects a second amount of the first light transmitted through the flow of plasma. This may be followed by emitting a second beam of the second light at the flow of plasma. The second photosensor detects a second amount of the second light transmitted through the flow of plasma. A current ratio of the second amount of the first light to the second amount of the second light is determined. Finally, a difference (in some embodiments the absolute value of the difference) between the baseline ratio and the current ratio is determined to exceed a predetermined amount. The first valve is then closed to stop the flow of plasma into the first collection bag. A second valve may then be opened to allow a flow of white blood cells into a second collection bag.

Further, an apparatus is provided for separating components from whole blood according to claim 12. In embodiments, the apparatus comprises a rotor having at least one separation cell. The at least one separation cell being configured to receive a separation bag containing a composite fluid. The apparatus includes at least one collection cavity that is configured to receive at least a first collection bag and a second collection bag. The apparatus further includes a first sensor and a second sensor for detecting light. Additionally, the apparatus includes a first light source, a second light source; a first valve for controlling flow into a first collection bag, and a second valve for controlling flow into the second collection bag. The apparatus includes at least one processor for executing processor readable instructions that when executed by the at least one processor performs the steps of any one of claims 1-11.

This summary is provided to introduce aspects of some embodiments of the present invention in a simplified form, and is not intended to identify key or essential elements of the claimed invention, nor is it intended to limit the scope of the claims.

Non-limiting and non-exhaustive embodiments are described with reference to the following figures.
FIG. 1 illustrates a first set of bags designed for use with a separation apparatus, according to an embodiment.
FIG. 2 illustrates a separation apparatus, partly in cross-section along a diametric plane, according to an embodiment.
FIG. 3 illustrates a top view of the separation apparatus of FIG. 2, showing at least part of a set of bags mounted thereon, valves, and sensors.
FIG. 4A and 4B illustrate light sources and sensors for detecting components in a tube, disposed in a path, according to an embodiment.
FIG. 5 illustrates another embodiment of light sources and sensors for detecting components in a tube, according to an embodiment.
FIG. 6 illustrates a flow chart of a process for controlling flow of a component, according to an embodiment.
FIG. 7-7E illustrate a flow chart of a process for controlling flow of whole blood components from a separation bag in which whole blood has been separated.
FIG. 8 illustrates a computer system, including a processor, in which embodiments may be implemented.

The principles of the present invention may be further understood by reference to the following detailed description and the embodiments depicted in the accompanying drawings. It should be understood that although specific features are shown in the figures and described below with respect to detailed embodiments, the present invention is not limited to these embodiments.

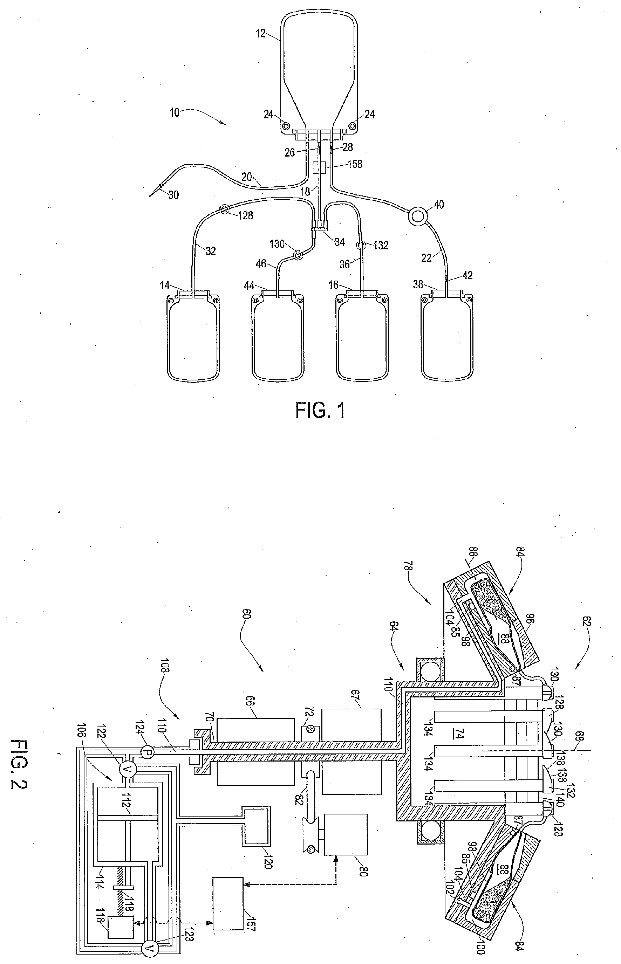

FIG. 1 illustrates an embodiment of a set 10 of bags adapted for use in separating a composite fluid (e.g. whole blood) into at least a first component and a second component (e.g. plasma, platelets, white blood cells, or red blood cells). The set 10 comprises a flexible separation bag 12 and flexible component or satellite bags 14, 16, 38 and 44, connected thereto. In other embodiments, the set 10 may comprise other type of containers, such as bottles, vials, or other containers with less flexible sides. Specific features of set 10 will be described below with respect to separation of whole blood into blood components; however it is noted that this is done merely for illustrative purposes, and the set 10 may be used in the process of separating other composite fluids.

Set 10 is shown with an asymmetrical manifold 34, which in this embodiment has an E shape. The manifold 34 may have other configurations with a number of different arms or connectors depending on the type of separation process and number of components being separated.

When the composite fluid being separated is whole blood, embodiments provide for the separation bag 12 to be used as a collection bag and as a separation bag. For example, in one embodiment, bag 12 is used initially to receive a discrete volume of whole blood from a donor (e.g., about 500 ml, such as between about 350 ml and about 600 ml) to be used later as a separation chamber in a separation apparatus. The separation bag 12 may be flat and generally rectangular. It may be made of two sheets of plastic material that are welded together so as to define there between an interior space having a main rectangular portion connected to a triangular proximal portion. A first tube 18 may be connected to a proximal end of the triangular portion, and a second tube 20 and a third tube 22 may be connected on sides adjacent the first tube 18. The proximal ends of the three tubes 18, 20, 22 may be embedded between the two sheets of plastic material so as to be substantially parallel. The separation bag 12 may further, optionally, include holes 24 in each of its two proximal corners that are adjacent to the three tubes 18, 20, 22. The holes 24 may be used to optionally secure the separation bag 12 to a separation cell on a separation apparatus, e.g., a centrifugal blood separation apparatus.

In embodiments, the separation bag 12 may initially contain a volume of anti-coagulant solution (for example, a solution of citrate phosphate dextrose). The first and third tubes 18, 22 may be fitted at their proximal ends with a stopper 26, 28 respectively, blocking liquid flow there through. The stopper 26 and 28 is in some embodiments a frangible. The second tube 20 may be a collection tube with a needle 30 connected to its distal end. At the beginning of a blood donation, the needle 30 may be inserted in the vein of a donor, causing blood to flow into the separation bag 12. After a desired volume of blood has been collected in the separation bag 12, the collection tube 20 may be sealed and cut, disconnecting the needle 30 from the bag set 10. Alternatively, in some embodiments, previously collected blood may be transferred to the separation bag 12 through the collection tube 20, with or without the use of the needle 30.

The first component bag 14 may be used to receive a plasma component. The bag 14, in this embodiment, may be flat and substantially rectangular. It may be connected, in some embodiments, through a plasma collection tube 32 and an asymmetric manifold 34 to the first tube 18. The second component bag 16 may receive a platelet component. The second component bag 16 may also be flat and substantially rectangular, and be connected through a platelet collection tube 36 and the asymmetric manifold 34 to the first tube 18. A third component bag 44 may be used to receive a third component, such as a white blood cell component from the primary bag 12. White blood cells may be drained through tube 46 and into bag 44.

A component bag 38 may receive red blood cells that are drained through tube 22, which may include a filter 40, into fourth component bag 38. A stopper 42, or frangible, in tube 22 as well as frangible 28 prevents premature flow of red blood cells into the fourth component bag 38.

In some embodiments, an optional wash solution bag (not shown), may be used in the set 10. In these embodiments, the wash solution bag may initially contain wash solution such as saline or a storage solution such as SAGM if no washing is desired. Wash solution may be transferred through a wash solution tube and the asymmetrical manifold 34 by way of the first tube 18 into the primary bag 12 when the primary bag 12 contains high hematocrit blood cells. "High hematocrit" means a percentage of red blood cell volume to total fluid volume of, in some embodiments, at least about 80 percent, at least about 90 percent, or at least about 95 percent. After wash solution is mixed with high hematocrit red blood cells and subsequently separated, used wash solution may be extracted through the first tube 18, asymmetrical manifold 34, and into a wash solution discard bag, which in some embodiments is the same as the wash solution bag. The discard bag could also be used to collect other components, such as for example, mesenchymal stem cells, or white blood cells.

FIG. 2 shows an embodiment of an apparatus 60 for simultaneously separating four discrete volumes of a composite fluid. Apparatus 60 separates the four discrete volumes of composite fluid by centrifugation. In other embodiments, the separation may be performed using a different process, non-limiting examples including vibration, filtration, or combinations of processes.

In the embodiment of FIG. 2, the apparatus 60 comprises a centrifuge 62 adapted to receive four sets of containers, for example, four of the sets 10 of bags shown in FIG. 1, with the four discrete volumes of a composite fluid contained in the four primary separation bags 12. The apparatus 60 may further include a component transferring system for transferring at least one separated component from each separation bag into a component bag connected thereto. The apparatus 60 may further include a system for washing a residual high hematocrit red blood cell component.

In the embodiment of FIG. 2, the centrifuge 62 comprises a rotor 64 that is supported by a bearing assembly 67 allowing the rotor 64 to rotate around a rotation axis 68. The rotor 64 includes a cylindrical rotor shaft 70 to which a pulley 72 is connected. A storage system, in the apparatus 60, includes a central cylindrical container 74 for containing component bags that is connected to the rotor shaft 70 at the upper end thereof so that the longitudinal axis of the rotor shaft 70 and the longitudinal axis of the container 74 coincide with the rotation axis 68. Four separation cells 78 are coupled to the central container 74 so as to form a symmetrical arrangement with respect to the rotation axis 68. The centrifuge further comprises a motor 80 coupled to the rotor by a belt 82 engaged in a groove of the pulley 72 so as to rotate the rotor about the rotation axis 68.

In embodiments, each separation cell 78 may have a container 84 having the general shape of a rectangular parallelepiped. The separation cells 78 may be mounted on the central container 74 so that their respective median longitudinal axes 86 intersect the rotation axis 68, so that they are located substantially at the same distance from the rotation axis 68, and so that the angles between their median longitudinal axes 86 are substantially the same (i.e. 90 degrees). The median axes 86 of the separation cells 78 are inclined downwardly with respect to a plane perpendicular to the rotation axis 68.

Each container 84 may include a separation cavity 88 that is so shaped and dimensioned as to loosely accommodate a separation bag 12 (FIG. 1) containing a composite fluid. The cavity 88 (which may be referred to also as the "separation compartment") is defined by a bottom wall, which is the farthest to the rotation axis 68, a lower wall that is the closest to the container 74, an upper wall opposite to the lower wall, and two lateral walls. The cavity 88 comprises a main part, extending from the bottom wall, which in embodiments has substantially the shape of a rectangular parallelepiped with rounded corners and edges, and an upper, or proximal, part, which has substantially the shape of a prism having convergent triangular bases. The upper part of the cavity 88 may be defined by two sets of two opposing walls converging towards the central median axis 86 of the container 84. Embodiments that include this design for cavity 88, provide for a radial dilatation of a thin layer of a component of a composite fluid (e.g. the platelets in whole blood) after separation by centrifugation, and makes the layer more easily detectable in the upper part of a separation bag. Also, in embodiments, this design reduces mixing between component layers by providing a gradual, funnel-like transition into the tube. The two sets of opposite walls of the upper part of the separation cell 78 converge towards three cylindrical parallel channels (not shown), opening at the top of the container 84, and through which, when a separation bag 12 is set in the container 84, the three tubes 18, 20, 22 extend. (Tube 20 along with needle 30 may be removed before the separation bag 12 is placed in the container 84).

The container 84 may also comprise a hinged lateral lid 96. In some embodiments, the lid 96 may be comprised of an upper portion of the external wall of the container 84. The lid 96 may be dimensioned to allow, when open, an easy loading of a separation bag 12 full of liquid into the separation cell 78. The container 84 may include a locking means (not shown) by which the lid 96 can be locked to the remaining part of the container 84. The container 84 may also comprise a securing or locating means for securing or locating a separation bag 12 within the separation cell 78. The bag securing or locating means may in some embodiments include two pins (not shown) protruding on the internal surface of the lid 96, close to the top of separation cell 78, and two corresponding recesses in the upper part of the container 84. The two pins may be spaced apart and dimensioned to fit into the two holes 24 in the upper corners of a separation bag 12.

In embodiments, the separation apparatus further comprises a component transferring means for transferring at least one separated component from each separation bag into a component bag connected thereto. The component transferring means may include a squeezing system for squeezing the separation bags 12 within the separation compartments 88 and causing the transfer of separated components into component bags 14, 16, 38, 44. The squeezing system in embodiments includes a flexible diaphragm 98 that is secured to each container 84 so as to define an expandable chamber 100 in the cavity 88 thereof. More specifically, the diaphragm 98 may be dimensioned so as to line the bottom wall of the cavity 88 and a large portion of the lower wall of the cavity 88. The squeezing system may further comprise a peripheral circular manifold 102 that may form a ring. In this embodiment, each expansion chamber 100 is connected to the manifold 102 by a supply channel 104 that extends through the wall of the respective container 84, close to the bottom thereof. The squeezing system may further comprise a hydraulic pumping station 106 for pumping a hydraulic liquid in and out of the expandable chambers 100 within the separation cells 78. The hydraulic liquid may be selected so as to have a density slightly higher than the density of the densest of the components in the composite liquid to be separated (e.g. the red blood cells, when the composite liquid comprises whole blood). As a result, during centrifugation, the hydraulic liquid within the expandable chambers 100, whatever the volume thereof, will in embodiments generally remain in the most external part of the separation cells 78. The pumping station 106 may be connected to the expandable chambers 100, through a rotary seal 108, by a duct 110 that extends through the rotor shaft 70, through the bottom and lateral wall of the central container 74, and, radially outwardly where it may connect to the manifold 102. The pumping station 106 comprises a piston pump having a piston 112 movable in a hydraulic cylinder 114 fluidly connected via the rotary seal or fluid coupling 108 to the rotor duct 110. The piston 112 may be actuated by a brushless DC motor 116 that moves a lead screw 118 linked to a piston rod. The hydraulic cylinder 114 may also be connected to a hydraulic liquid reservoir 120 having an access controlled by two valves 122, 123 for selectively allowing the introduction or the withdrawal of hydraulic liquid into and from a reciprocating hydraulic circuit including the hydraulic cylinder 114, the rotor duct 110 and the expandable hydraulic chambers 100. A pressure gauge 124 may be connected to the hydraulic circuit for measuring the hydraulic pressure therein.

The container 84 may further comprise a pressure sensor 85 in the wall of the cavity 88. The pressure sensor 85 may have a flexible membrane that moves in response to pressure changes in cavity 88 and such membrane may be connected to a pressure transducer to convert such information to pressure information. In embodiments, the pressure sensor 85 senses pressure amount due to the fluid level or head height of fluid in the bag 12. The outboard area of the cavity 88 may extend from the sensor 85 to the bottom wall or the wall furthest from the rotational axis. The inboard area of the cavity 88 may be an area from the sensor 85 to the upper wall of the cavity 88. The container 84 may further include a second optical sensor 87 in the wall of the cavity 88 to detect passage of blood component or interface changes as more fully described below.

The separation apparatus may further comprise four sets of three pinch valves 128, 130, 132 that are mounted on the rotor 64 around the opening of the central container 74. Each set of pinch valves 128, 130, 132 may face one separation cell 78, with which it is associated. The pinch valves 128, 130, 132 are designed for selectively blocking or allowing a flow of liquid through a flexible plastic tube, and selectively sealing and cutting a plastic tube. Each pinch valve 128, 130, 132 may comprise an elongated cylindrical body 134 and a head 136 having a jaw 138 forming a gap that is defined by a stationary lower plate or anvil 140 and the jaw 138 movable between a "load" position, an "open" position, and a "closed" position. The gap may be so dimensioned that one of the tubes 22, 32, 36, 46 of the bag sets 10 shown in FIG. 1 can be snuggly engaged therein when the jaw 138 is in the open position. In embodiments, the elongated body 134 contains a mechanism for moving the jaw 138 and it may be connected to a radio frequency generator that supplies the energy necessary for sealing and cutting a plastic tube. It is also noted that in other embodiments a tearable seal could alternatively be used. The pinch valves 128, 130, 132 may be mounted inside the central container 74, adjacent the interior surface thereof, so that their longitudinal axes may be parallel to the rotation axis 68 and their heads protrude above the rim of the container 74. An embodiment of a position of a set of pinch valves 128, 130, 132 with respect to a separation bag 12 and the tubes 32, 36, 46 connected thereto when the separation bag 12 rests in the separation cell 78 associated with this set of pinch valves 128, 130, 132 is shown in dotted lines in FIG. 1. Electric power may be supplied to the pinch valves 128, 130, 132 through a slip ring array 66 that is mounted around a lower portion of the rotor shaft 70.

The separation apparatus 60 may also comprise a controller 157 including a control unit (e.g. a processor or other integrated circuit) and a memory unit for providing the processor with information and programmed instructions relative to various separation protocols (e.g. a protocol for the separation of a plasma component and a blood cell component, or a protocol for the separation of a plasma component, a platelet component, and a red blood cell component) and to the operation of the apparatus 60 in accordance with such separation protocols. In particular, the processor may be programmed for receiving information relative to the centrifugation speed(s) at which the rotor 64 is to be rotated during the various stages of a separation process (e.g. stage of component separation, stage of a plasma component expression, stage of suspension of platelets in a plasma fraction, stage of a platelet component expression, etc), and information relative to the various transfer flow rates at which separated components are to be transferred from the separation bag 12 into the component bags 14, 16. The information relative to the various transfer flow rates can be expressed, for example, as hydraulic liquid flow rates in the hydraulic circuit, or as rotation speeds of the brushless DC motor 116 of the hydraulic pumping station 106. The processor may be further programmed for receiving, directly or through the memory, information from the pressure sensor 124 and from the sets of sensors 87 and 158 (described below) and for controlling the centrifuge motor 80, the brushless DC motor 116 of the pumping station 106, and the four sets of pinch valves 128, 130, 132 so as to cause the separation apparatus 60 to operate along a selected separation protocol. The processor may also receive information from each pressure sensor 85 and sensors 87 and 158 for volume determination or prediction, and for other determinations as described in greater detail below.

A first balancing means may initially balance the rotor when the weights of the four separation bags 12 contained in the separation cells 78 are different. The first balancing means in embodiments comprises similar structural elements as the elements of the component transferring means described above, namely: four expandable hydraulic chambers 100 interconnected by a peripheral circular manifold 102, and a hydraulic liquid pumping station 106 for pumping hydraulic liquid into the hydraulic chambers 100 through a rotor duct 110, which is connected to the circular manifold 102. Under centrifugation forces, the hydraulic liquid will distribute unevenly in the four separation cells 78 depending on the difference in weight of the separation bags 12 to balance the rotor 64.

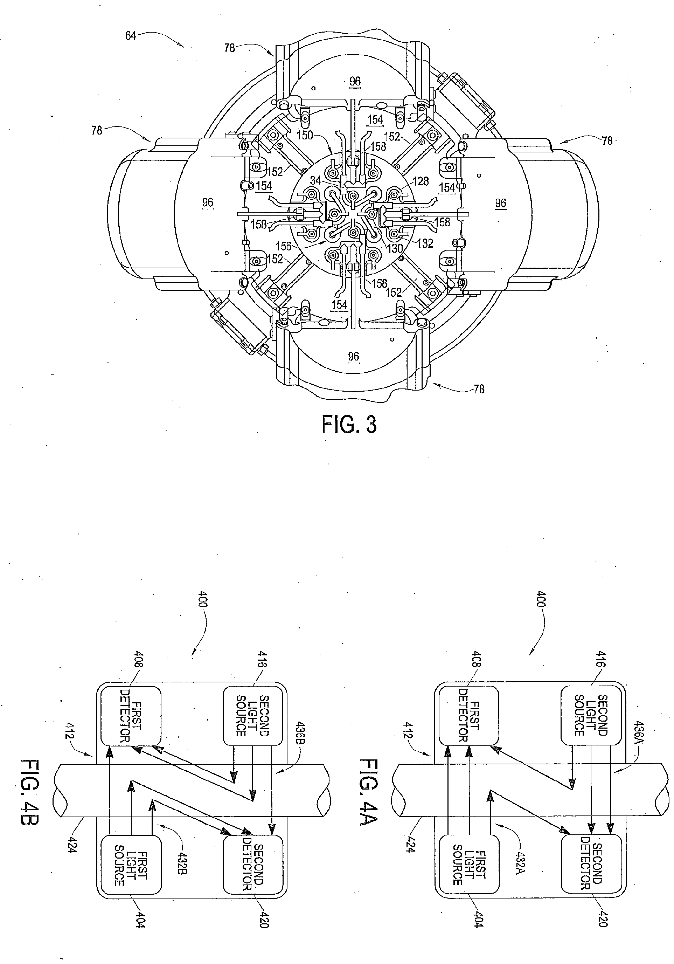

FIG. 3 shows a top plan view of an embodiment of the rotor 64. Four symmetrically spaced separation cells 78 (each with a lid 96) are shown surrounding a central core 150, which contains four sets of valves 128, 130, 132 and which supports the asymmetrical manifolds 34 and tubes of the bag sets 10. The core 150 may be supported in the center of the rotor by a spider structure comprised of four radial support arms 152. The arms 152 may define collection cavities 154 between a separation cell 78 and an adjacent set of valves 128, 130, 132 on the central core 150. The component bags 14 and 16 (for plasma and platelets respectively) and the red blood cell component bag 38, with its associated filter 40, may be placed in the cavity 154 when the bag set 10 is loaded into the rotor 64. The collection and separation bag 12, which may initially contain a collected unit of whole blood, may be placed in the adjacent separation cell 78. The auxiliary bag 44, which may be used for temporary fluid storage, waste fluid collection or collection of a rare or small-volume blood component, may be placed in a well 156 close to the axis of rotation 68 (see FIG. 3) of the rotor. The well 156 may be closer to the axis of rotation than at least some of the valves associated with a single set 10 of bags. The well 156 may be cylindrical or rectangular to accommodate a rectangular bag 44, as shown in FIG. 1. The well 156 may be positioned such that the processing or primary separation bag 12 is located in a relatively high force region of the centrifugal field produced by the rotation of the rotor 64, while the component bags 14, 16 may be located in a lower force region, and the smaller wash solution or discard bag 44 may be placed in the well would be in the lowest force region. By reason of bag placement in high, intermediate and low force regions of the centrifugal field, in embodiments, air will tend to collect in the wash bag 44 in the well 156. Moreover, a shorter line or tube can be used to connect the small bag 44 to the entire bag assembly. The three placement zones, in embodiments, aid in simplifying the bag assembly and make the process of loading the bag assembly into the rotor easier.

FIG. 3 also shows an embodiment of an asymmetric manifold 34 having an "E" configuration (also shown in FIG. 1), although other configurations could be used. For each set of valves, outer valves 128, 132 are shown in "load" configuration, that is, the jaw 138 of the valve does not extend over an adjacent tube, thereby allowing the manifold 34 and tubes to be installed in their proper configuration on the central core 150. For each set of valves, an inner or center valve 130 is shown in the closed position with respect to tube 46.

A tube sensor 158 is used in some embodiments to detect the presence or absence of liquid in the tube 18, such as the detection of plasma, as well as to detect blood cells in a liquid in other embodiments. Each sensor 158 and 87 (FIG. 2) may, in embodiments, comprise a photocell with an LED (e.g., infrared, red, blue, green, or other color light) and a photo-detector. Electric power may be supplied to the sensors 87 and 158 through the slip ring array that is mounted around the lower portion of the rotor shaft 70. In the process of separating blood into component parts, fluid components, such as plasma or platelets, may be expressed out of the separation bag 12 in the separation cell 78 into component bags 14, 16 in the cavities 154. The sensor 158 may detect the beginning of the plasma flow or the presence of platelets or red blood cells. In response, the controller 157 may interrupt or change the processing for the particular set of bags where the new condition was sensed. The second sensor or photocell 87, similar to 158, may also be included in container 84 (see FIG. 2). The sensor 87 may also be able to detect the presence or absence of liquid such as plasma as well as the presence of platelets or red blood cells. This sensor may be used to detect the trailing edge of a separated layer or fraction.

Embodiments provide for separating composite fluid or whole blood into components. Examples of some embodiments for separating whole blood into components will be described below. Embodiments begin with collection of the whole blood or fluid into a container such as bag 12 of bag set 10 (FIG. 1). The bag set 10 may then be loaded onto apparatus 60. Before loading the bag set 10, needle 30 and tube 20 may be removed by sterile welding or other procedure. The bag 12 containing the composite fluid or whole blood may be placed in the cavity 88. A different bag set 10 with a bag 12 containing the composite fluid or whole blood may be place in each cavity 88 of the apparatus 60. The collection bags and other fluid bag 14, 16, 38, 44, if any, may be placed in the respective cavity 154 or 156.

The rotation of the rotor 64 begins and the rotor 64 is rotated until it reaches an rpm suitable for separation, for example, in some embodiments, the rpm's may range from about 1800 rpm to about 3200 rpm. A pressure sensing value at a designated rpm may be provided by pressure sensor 85 to the controller 157. Pressure sensor 85 senses the fluid pressure in the cavity 88 due to the head height or fluid level of the composite fluid. The valves may be opened or closed during the pressure sensing step. Pressure may be determined also after loading of bag 12 but before the rotor 64 begins its rotation. The fluid pressure measurement may be used by the controller to predict the volume of the composite fluid or whole blood. The pressure amount corresponds to the fluid level or head height, in embodiments, and thus corresponds to the composite fluid volume.

During centrifugation, fluid in the separation bag 12 is subject to a radial acceleration gradient. This causes a pressure gradient along a radial line passing through the fluid volume. The resulting pressure depends on the angular velocity, radius and fluid density.

Pressure sensor 85 measures the fluid pressure at a known radial location in the separation bag 12. The rotor speed or rpm of the rotor 64 is known as is the radial location of the pressure sensor 85 on the centrifuge 62. The sensor 85 is further located at a specific location along the wall of the separation compartment 88. A portion of the composite liquid will be outboard or between the sensor 85 and the bottom of the separation compartment 88. The volume of composite fluid can be determined from the volume of the outboard area containing such fluid. Fluid variations may occur in the inboard area of the separation compartment 88 due to the overall volume of the composite fluid. Larger volumes of fluid may cause more fluid to be located in the inboard area or the area between the sensor 85 and the top of the separation compartment 88. As fluid volume increases inboard of the pressure sensor 85 the magnitude of the pressure at the sensor 85 location may also increase. The radial distance from the fluid surface to the pressure sensor 85 location can be determined. The volume of the outboard area is known from the separation compartment 88 geometry and the volume of the inboard area can be estimated by variations in pressure due to the pressure sensor 85. Adding the outboard volume to the inboard volume provides an estimate or prediction of the total volume of the composite fluid.

As rotation continues, in embodiments, valve 128 opens or remains open and plasma, the least dense component in whole blood, flows into bag 14. The hydraulic fluid from reservoir 120 may flow through duct 110 and channel 104 and under bladder or diaphragm 98 to squeeze bag 12 to facilitate plasma transfer to bag 14. Photocell 158 may optically sense the leading edge of the plasma flow and may provide such information to controller 157. When photocell or sensor 87 senses a cellular component approaching tubing 18 from the top of bag 12, it may also provide the information including the trailing edge of the plasma interface to the controller 157. The controller 157 may send a signal to close valve 128 and open valve 132 for cellular component. The hydraulic flow rate corresponds to the fluid flow rate into the collection bag 14 and 16. From this flow rate and the sensor signals 87, 158 determining the start (leading edge) and end (trailing edge) of the plasma collection, the volume of plasma or an expressed component can be determined.

Sensors 87 and 158 further can sense the change of cellular component from, for example, platelets to red blood cells. Sensor 158 may sense the leading edge of the platelet layer with sensor 87 indicating the trailing edge. This sensing of an interface change between different layers or sedimented blood components may cause the controller to signal valve 132 to close and end the platelet collection. The sensor 158 may send a signal indicating platelets, and sensor 87 may send a signal indicating the end of the platelet layer to the controller, along with the fluid flow rate, to determine or estimate the volume of platelets transferred. The estimated plasma volume as well as the determined platelet volume along with the initial whole blood volume estimate can be used by the controller 157 to provide an estimate of the remaining component or components such as red blood cells in bag 12.

If it is desired to add wash solution or storage solution into the remaining red blood cells, the hydraulics 112 may be pulled back to drain out from under diaphragm or membrane 98 to release the squeezing pressure on the previously squeezed bag 12. Valve 130 may be opened and wash or storage solution may be introduced from bag 44 through tubing 46 to bag 12.

If washing is desired, the wash solution may be mixed with the red blood cells and resulting supernatant can be expressed back to bag 44 using the hydraulic fluid to squeeze bag 12 for the transfer. If storage solution is added from bag 44, the centrifuge may be stopped. The bag set 10 may be removed from the centrifuge and all tubing but 22 can be discarded. Storage solution can be also gravity drained from bag 38 through filter 40 to mix with remaining red blood cells in bag 12.

Valves 128, 130 and 132 can provide heat sealing of the tubing 32, 46 and 36 in some embodiments. Any remaining tubing can also be heat sealed by the operator for removal leaving bag 12, tubing 22, filter 40 and bag 38 remaining. The residual product, such as red blood cells and storage solution, may be drained from bag 12 to 38. Frangibles 28 and 42 may be opened and bag 12, may be elevated to gravity drain the red blood cells through leukoreduction filter 40 to bag 38.

FIGS. 4A and 4B illustrate an embodiment of a sensor 400 that may be used as sensor 158 and/or sensor 87 (FIGS. 2 and 3). In the embodiment shown in FIGS. 4A and 4B, the sensor 400 includes two different light sources 404 and 416, each which emits a light of a different wavelength, and two detectors 408 and 420 that detect light of at least the two different wavelengths emitted by the light sources 404 and 416. As shown in FIG. 4, a first light source 404 is located across a path 412 from first detector 408. In the embodiment of FIG. 4, first detector 408 is positioned substantially opposite first light source 404. A second light source 416 is located across path 412 from second detector 420. Second detector 420 is positioned substantially opposite second light source 416. As illustrated in FIGS. 4A and 4B, a conduit, such as tubing 424, is positioned in path 412. In the embodiments shown in FIGS. 4A and 4B, a flow of fluid is passing through tubing 424. In embodiments, the flow may be a flow one or more components separated from a composite fluid (e.g., blood). Tubing 424, in embodiments, is made from material that is at least translucent and in some embodiments substantially transparent to light generated from light sources 404 and 416.

In one embodiment of sensor 400, first light source 404 may be activated to generate beams 432A (FIG. 4A), 432B (FIG. 4B) of a first light that is directed toward first detector 408. As noted above, tubing 424 may be substantially transparent to the first light. Accordingly, most of the light in beams 432A, 432B may be transmitted through the tubing 424. Depending on the characteristics of the fluid flowing through tubing 424, an amount of the first light in beams 432A, 432B may be transmitted through the tubing 424 and the flow of fluid, and detected by the first detector 408. Another amount may be reflected by the flow of fluid in tubing 424 and detected by the second detector 420.

Similarly, the second light source 416 may be activated to generate beams 436A (FIG. 4A), 436B (FIG. 4B) of a second light that is directed toward second detector 420. As noted above, tubing 424 may be substantially transparent to the second light. Accordingly, most of the light in beams 436A and 436B may be transmitted through the tubing 424. Depending on the characteristics of the fluid flowing through tubing 424, an amount of the second light in beams 436A, 436B may be transmitted through the tubing and the flow of fluid, and detected by the second detector 420. Another amount may be reflected by the flow of fluid in tubing 424 and detected by the first detector 408.

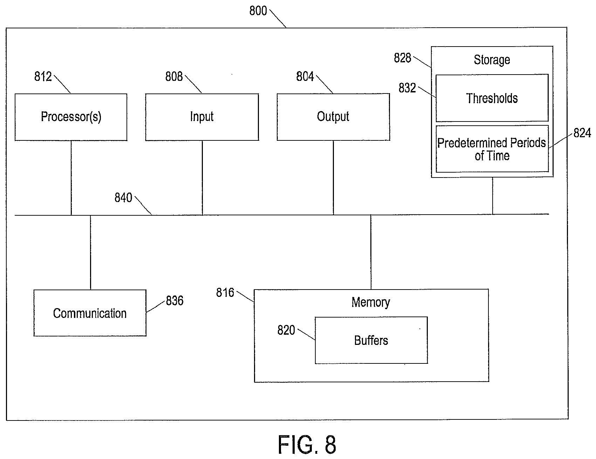

In embodiments, sensor 400 may be connected to a basic computer system, such as a controller, e.g., controller 157 (FIG. 2). The basic computer system may include a number of different components such as the components described with respect to computer system 800, including a processor such as processor 812, described below with respect to FIG. 8. The basic computer system may be a controller, or part of a controller, such as, for example, controller 157. Sensor 400 sends signals to a processor indicating the amount of light detected by each detector 408 and 420. In embodiments, the amounts may be used to determine at least one component in the flow passing through tubing 424. As described in greater detail below, the processor may use multiple measurements of detected light from the detectors 408 and 420 to make determinations. Also, in response to the determinations, the processor may open or close valves 128, 130, 132, (Fig. 2) used to direct components of the composite fluid into various containers.

In one embodiment, sensor 400 may be used to determine when a change of components (e.g., components of a composite liquid) has occurred in the flow passing through tubing 424. For example, sensor 400 may be used to determine when a component begins to flow in tubing 424. In this embodiment, as shown in FIG. 4A, first light source 404 is activated to generate beam 432A. First detector 408 then detects a first amount of light from beam 432A that is transmitted through the flow in tubing 424. Second light source 416 is activated to generate beam 436A. Second detector 420 then detects a first amount of light from beam 436A that is transmitted through the flow in tubing 424. In embodiments, a baseline ratio is then determined by obtaining the ratio of the first amount of the first light detected by first detector 408 to the first amount of the second light detected by the second detector 420.

As shown in FIG. 4B, after a period of time, first light source 404 may be activated again to generate a second beam 432B. First detector 408 then detects a second amount of light from beam 432B that is transmitted through the flow in tubing 424. Second light source 416 is activated to generate beam 436B. Second detector 420 then detects a second amount of light from beam 436B that is transmitted through the flow in tubing 424.

In embodiments, a current ratio is then determined by obtaining the ratio of the second amount of the first light detected by first detector 408 to the second amount of the second light detected by the second detector 420. A comparison is then made to determine whether the difference (in some embodiments the absolute value of the difference) between the current ratio and the baseline ratio meets a predetermined threshold. If the predetermined threshold is met, various actions, such as open and closing of valves (e.g. valves 128, 130, 132) or controlling the flow of hydraulic fluid in a squeezing system, for example, may be performed.

In embodiments, first light source 404 and second light source 416 are quickly activated "on" and "off." In these embodiments, detectors make readings each time the light sources are activated. Thus, the baseline ratio may be calculated when the light sources 404 and 416 are initially activated, and the current ratio can be calculated each subsequent time the light sources 404 and 416 are activated. In other embodiments, when the difference (in some embodiments the absolute value of the difference) between the baseline ratio and the current ratio meets a predetermined threshold, the baseline ratio may be recalculated using the information from the subsequent activation of the light sources 404 and 416 following the threshold being met.

As can be appreciated, in some embodiments, instead of determining the amount of light that is transmitted through the flow, detectors 408 and 420 may be used to detect the amount of light that is reflected by the flow in tubing 424. For example, referring to FIG. 4A, to determine a baseline ratio, detector420 may detect the amount of light from beam 432A that is reflected by the flow, and detector 408 may detect the amount of light from beam 436A that is reflected by the flow. Similarly, referring to FIG. 4B, to determine a current ratio, detector 420 may detect the amount of light from beam 432B that is reflected by the flow, and detector 408 may detect the amount of light from beam 436B that is reflected by the flow. The baseline ratio and the current ratio can be compared to determine whether the difference (in some embodiments the absolute value of the difference) in ratios meets a predetermined threshold, which as noted above can trigger the performance of various actions.

In the embodiment shown in FIG. 4B, the amount of light transmitted through the flow in tubing 424 is less than the amount transmitted through the flow as shown in FIG. 4A, i.e., at an earlier point in time. In embodiments, this is due to the characteristics of the flow in tubing 424. For example, the flow shown in FIG. 4B may have particles that reflect light, and therefore allow less light to be transmitted through the flow. Accordingly, the transmission and reflection of light can be used to determine the presence of particles that may not have been initially present in the flow. Alternatively, in other embodiments, the transmission of light may be lower at an earlier point in time, and greater at a later point in time, signaling that particles may no longer (or less of them) be in the flow in tubing 424.

In embodiments, the light generated from light sources 404 and 416 is different. For example, the light generated by source 404 may be of a different wavelength than the light generated from source 416. In embodiments, the light generated by source 404 may be of a smaller wavelength (or in other embodiments, larger wavelength) than the light generated by source 416. In embodiments, both light sources may generate light in the visible light spectrum, but one source may generate visible light of a shorter wavelength. In one embodiment, light source 404 generates blue visible light while light source 416 generates red visible light. In other embodiments, light sources 404 or 416 may generate violet, blue, green, yellow, orange, or red visible light, as some non-limiting examples.

The light sources 404 or 416 may be of any suitable type. Non-limiting examples of light sources 404 or 416 that may be used include, electroluminescent sources such as (light emitting diodes (LEDs), electroluminescent sheets, electroluminescent wires, field-induced polymer electroluminescent (FIPEL)); electron-stimulated sources; incandescent sources; and/or gas discharge sources. The detectors 404 and 420 may be any suitable detector for detecting light. Some examples of detectors that may be used as detectors 404 and 420 include, without limitation, active pixel sensors; charged-coupled devices; optical detectors; photoresistors; photodiodes; phototransistors; and/or quantum dot photoconductors/photodiodes.

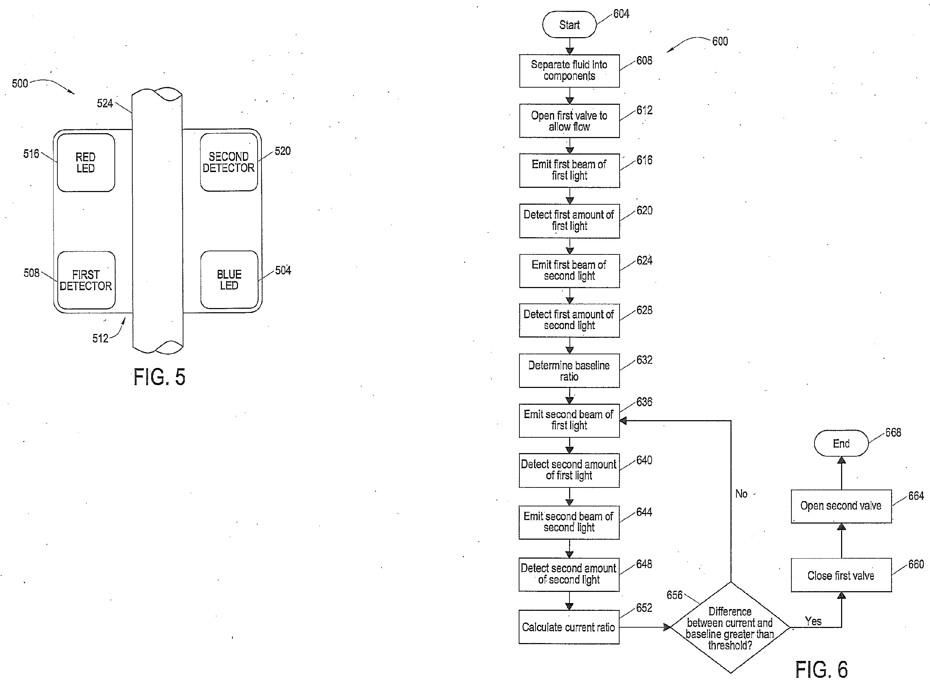

FIG. 5 illustrates an embodiment of a sensor 500 that may be used as sensor 158 and/or sensor 87 (FIGS. 2 and 3), and used in a system/apparatus 60 for separating a composite liquid such as blood into components. In the embodiment shown in FIG. 5, the sensor 500 includes two different light sources LED 504 and LED 516. LED 504 generally emits light of a wavelength within the blue light spectrum, such as from about 450 nanometers (nm) to about 495 nm. LED 516 generally emits light of a wavelength within the red light spectrum, such as from about 620 nm to about 750 nm. In other embodiments, LEDs 504 and 516 may emit light of other wavelengths.

Sensor 500 further includes two detectors 508 and 520 that detect light of at least the two different wavelengths emitted by the light sources 504 and 516, namely red and blue visible light for the embodiment shown in FIG. 5. As shown in FIG. 5, LED 504 is located across a path 512 from first detector 508. LED 516 is located across path 512 from second detector 520. A tubing 524 is positioned in path 512. Tubing 524 may, at points in time, have a flow of fluid passing through the interior of the tubing. The tubing 524 is substantially transparent to the light generated by the LEDs 504 and 516.

As noted above, in embodiments, whole blood is separated into components, and the components are removed from a separation bag through tubing 524. Accordingly, in embodiments, the flow in tubing 524 may include whole blood or various components of whole blood.

First detector 508 may be used to measure an amount of light from the LED 504 that is transmitted through the tubing 524 and flow in tubing 524, and also an amount of light from the LED 516 that may be reflected back by the flow in tubing 524. Second detector 520 may be used to measure an amount of light from the LED 516 that is transmitted through the tubing 524 and the flow in tubing 524, and also an amount of light from LED 504 that is reflected back by the flow in tubing 524.

In one embodiment, when LED 504 is activated both detectors 508 and 520 are read. LED 504 is then turned off, and both detectors 508 and 520 are read again. The LED 516 may then be activated, and again, both detectors 508 and 520 are read. LED 516 is then turned off, and both detectors 508 and 520 are read again. The detector readings taken when the LEDs 504 and 516 are not activated are subtracted from the detector readings when the LEDs 504 and 516 are activated to cancel out the effects of background light. In one embodiment, detectors 508 and 520 are read using a 12-bit analog to digital (A/D) converter.

In some embodiments, detectors 508 and 520 may be connected to an amplifier. The gain of the amplifier associated with the detectors 508 and 520 may be adjustable in some embodiments with an 8-bit digital potentiometer. As noted above, sensor 500 may be used as a sensor in a system/apparatus for separating whole blood into components, e.g., apparatus 60 (FIG. 1).

In one embodiment, sensor 500 is used to determine when a change of blood components has occurred in the flow passing through tubing 524. As described above, apparatus 60 may separate whole blood into components that include plasma, platelets, white blood cells, and red blood cells. In embodiments, sensor 500 may be used as line sensor 158 (FIG. 3) and be used to determine when a component (e.g., plasma, platelets, white blood cells, and red blood cells) begins to flow in tubing 524.

In embodiments, LED 504 is activated to emit blue light. Detector 508 detects a first amount of blue light that is transmitted through the flow in tubing 524. Detector 520 then detects a second amount of blue light that is reflected by the flow in tubing 524. LED 516 is then activated to emit red light. Detector 520 then detects a first amount of red light transmitted through the flow in tubing 524. Detector 508 then detects a second amount of red light that is reflected by the flow in tubing 524. In embodiments, a baseline ratio can then be determined by obtaining the ratio of the first amount of the blue light detected by detector 508 to the first amount of the red light detected by second detector 520. Alternatively, the baseline ratio may be calculated by obtaining the ratio of the second amount of the blue light detected by detector 520 to the second amount of the red light detected by the detector 508.

After a period of time, LED 504 is activated again to emit blue light. Detector 508 detects a third amount of blue light that is transmitted through the flow in tubing 524. Detector 520 then detects a fourth amount of blue light that is reflected by the flow in tubing 524. LED 516 is then activated to emit red light again. Detector 520 then detects a third amount of red light transmitted through the flow in tubing 524. Detector 508 then detects a fourth amount of red light that is reflected by the flow in tubing 524. A current ratio can then be determined by obtaining the ratio of the third amount of the blue light to the third amount of the red light. Alternatively, the current ratio may be calculated by obtaining the ratio of the fourth amount of the blue light detected by detector 520 to the fourth amount of the red light detected by the detector 508.

A comparison can then be made to determine whether the difference (in some embodiments the absolute value of the difference) in the current ratio and the baseline ratio meets a predetermined threshold. If the predetermined threshold is met, various actions, such as open and closing of valves (e.g. valves 128, 130, 132) or controlling the flow of hydraulic fluid in a squeezing system, for example, may be performed.

The difference (in some embodiments the absolute value of the difference) between the current ratio and baseline ratio is in embodiments an indication that different components of blood are passing through the tubing 524. For example, when a volume of whole blood has been separated into plasma, platelets, white blood cells, and red blood cells, plasma will be removed from a separation bag first. Accordingly, plasma will pass through tubing 524 before other components. The baseline ratio may be established, in some embodiments, when plasma is passing through tubing 524.

Plasma is typically relatively transparent to visible light and therefore when plasma is passing through tubing 524, a large amount of light (red and blue) will be transmitted through the plasma. After most of the plasma has been removed from the separation bag, platelets will begin to flow through tubing 524. Without being bound by theory, it is believed that when platelets are in the flow of fluid in tubing 524, they will scatter (e.g., reflect) light allowing less light (red and blue) to be transmitted through the flow. This can be determined by detector 508 receiving less blue light, when LED 504 is activated, and receiving more red light when LED 516 is activated. Similarly, because of the platelets scattering of light, detector 520 receives less red light, when LED 516 is activated, and receives more blue light when LED 504 is activated. The additional scattering of light can therefore be used to determine that platelets are in the flow in tubing 524.

After most of the platelets have been removed from the separation bag 12, white blood cells will begin to enter the flow in tubing 524. White blood cells are typically larger than platelets and therefore will scatter more light. Again, detectors 508 and 520 are used to determine that white blood cells are in the flow in tubing 524.

After most of the white blood cells have been removed from the separation bag 12, red blood cells will begin to enter the flow in tubing 524. Red blood cells typically absorb the visible light. Again, detectors 508 and 520 can be used to determine that red blood cells are in the flow in tubing 524.

Different actions can be initiated when sensor 500 detects the presence of different components in tubing 524. For example, in one embodiment, each time a different component is sensed by sensor 500, a valve to allow the flow to enter into a container designated for collecting a specific component can be opened, while other valves are closed.

In some separation procedures, it is desirable to collect platelets with as few white blood cells as possible. Accordingly, sensor 500 can be used to generate a volume of platelets with less white blood cells than is typically collected using conventional systems. The use of two light sources of different wavelengths (e.g., LED 504 and LED 516) allows the presence of white blood cells to be detected more quickly than with conventional sensors. Being able to detect the presence of white blood cells allows the white blood cells to be diverted from a container holding the volume of platelets to a different container, generating a volume of platelets with a relatively low concentration of white blood cells.

It is believed that the use of two different wavelengths of light, and a determination of when a ratio of the amount of the two different wavelengths (e.g., blue light and red light) of light transmitted through, or reflected by, a flow, provides a recognition of the presence of white blood cells more quickly. Without being bound by theory, it is believed that smaller wavelengths of light will be reflected by white blood cells before relatively larger wavelengths of light. Accordingly, a ratio of the light transmitted through, or light reflected by, the flow when both plasma and platelets are in the flow, e.g., a baseline ratio, and determining when a current ratio begins to differ from the baseline ratio by a predetermined threshold value, indicates that white blood cell are present in the flow. Appropriate actions can then be taken such as directing the flow to a different container to ensure that white blood cells are not added to a volume of platelets that have been collected. Sensor 500 may be used in embodiments to implement a procedure for recognizing the presence of white blood cells quickly, and collecting a volume of platelets with relatively few white blood cells.

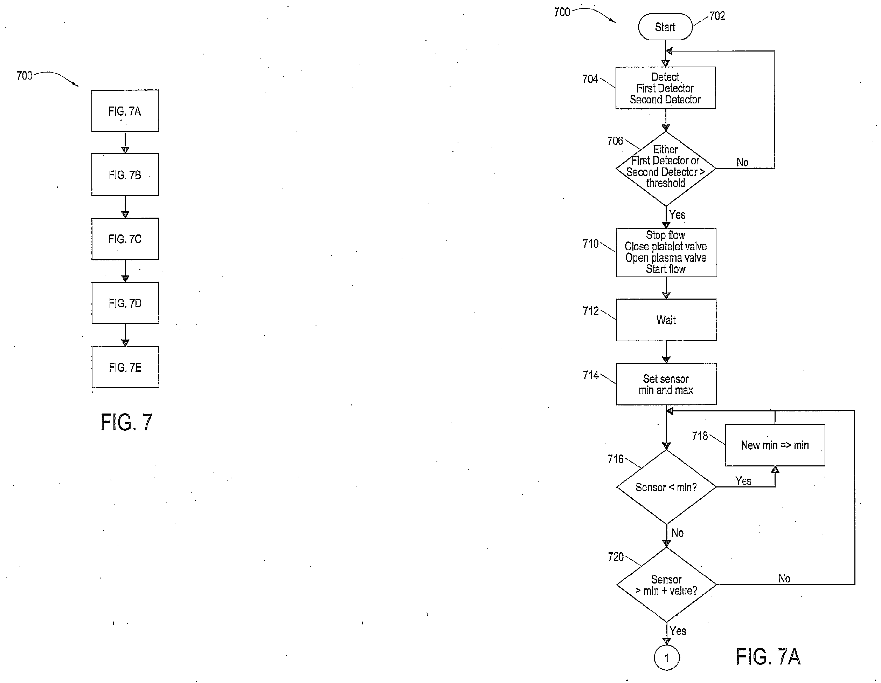

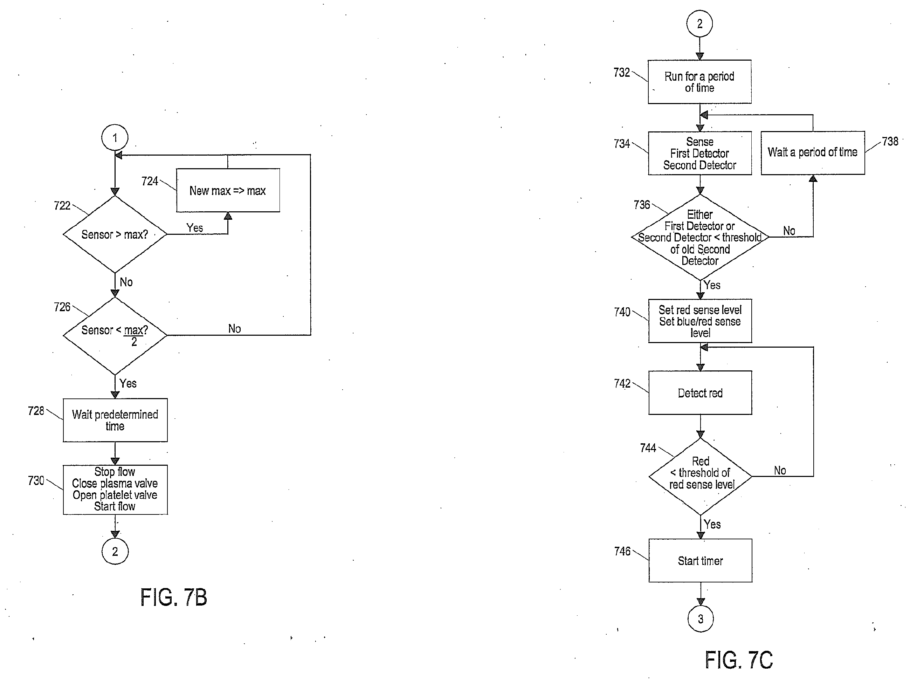

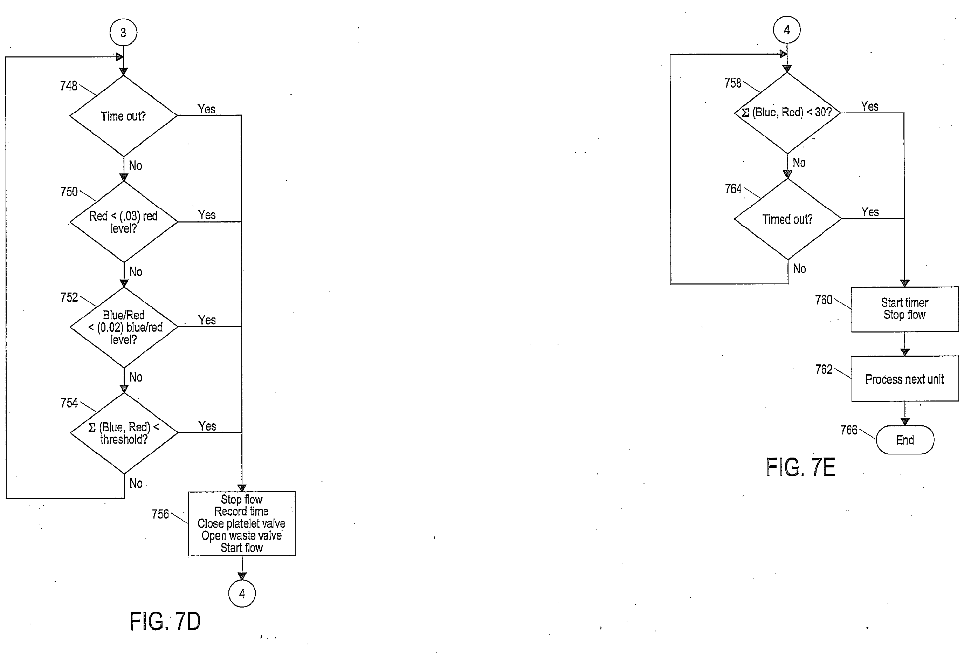

FIGS. 6 and 7 illustrate flow charts 600 and 700 that may be performed in embodiments of the present invention. Although specific structures, devices, or parts may be described below for performing steps in flow charts 600 and 700, the present invention is not limited thereto. For example, some steps may be described as performed by structures, devices, or parts of a sensor, such as sensor 87 (FIG. 2), 158 (FIG. 3), 400 (FIG. 4), or 500 (FIG. 5), while others may be described as performed by a processor or controller. This is done merely for illustrative purposes, and flow charts 600 and 700 are not limited to being performed by any specific structure, device, or part.

FIG. 6 illustrates a flow chart 600 for a process of controlling a flow that includes a component separated from a composite liquid. For example, in some embodiments, the composite liquid may be whole blood and the component may be one or more of plasma, platelets, white blood cells, red blood cells, or combinations thereof. Flow chart 600 starts at 604. Flow chart 600 proceeds from 604 to optional step 608, where the composite fluid is separated into components. In embodiments, step 608 may be performed by an apparatus such as apparatus 60 (FIG. 2). In other embodiments, step 608 may be performed by different systems that may utilize different technologies for separating components from a composite fluid, including without limitation, centrifugation, vibration, filtration, and combinations thereof. In one embodiment, step 608 is performed using an embodiment of apparatus 60 (Fig. 2).

From step 608, flow chart 600 passes to step 612 where a first valve is open to allow a flow of fluid that includes a component of the composite fluid into a container for collecting a volume of the component. In those embodiments where the composite fluid is blood, the flow of fluid may originate from a separation container, e.g., separation bag 12 (Fig. 1), and when the valve is opened at step 612, the flow of fluid is directed to a collection container, e.g., component bag 14 (Fig. 1). In one specific embodiment, the flow of fluid may comprise platelets and plasma.

After step 612, a first beam of a first light is emitted at the flow of fluid at step 616. In embodiments, step 616 may be performed by activating a light source, which is positioned so that when the first beam of the first light is emitted it is directed at the flow of fluid, which may be flowing through a conduit, such as tubing. As can be appreciated, the tubing through which the flow of fluid is traveling may be substantially transparent to the first light. In other embodiments, step 616 may involve activating a light source, and directing the first beam of the first light so that it is directed at the flow of fluid.

The light source used to emit the first beam of the first light at step 616 may be of any suitable type. In some embodiments, the light source is one or more of the light sources 404 and 416, described above with respect to FIG. 4. The light sources that may be used include, electroluminescent sources such as (light emitting diodes (LEDs), electroluminescent sheets, electroluminescent wires, field-induced polymer electroluminescent (FIPEL)); electron-stimulated sources; incandescent sources; and/or gas discharge sources. Accordingly, in embodiments, the first light source may be one or more of LED's 504 and 516 (FIG. 5). In some embodiments, step 616 may involve activating a light source to generate the first beam of the first light, and directing the first beam of the first light to the flow of fluid using light transmission methods/devices, some non-limiting examples including, fiber optics, reflectors (e.g., mirrors), or lenses.

Following step 616, a first amount of the first light is detected at step 620. In embodiments, the first amount of the first light may be an amount of the first light that is transmitted through the flow of fluid, while in other embodiments the first amount of the first light may be an amount of light that is reflected by the flow of fluid. In yet other embodiments, step 620 may involve detecting both the amount of the first light that is transmitted through the flow of fluid, and the amount of the first light that is reflected by the flow of fluid.

In embodiments, step 620 is performed by one or more light detectors. In embodiments, step 620 may be performed using one or more of detectors 408 and 420 (FIG. 4). Some examples of detectors that may be used in performing step 620 include, without limitation, active pixel sensors; charged-coupled devices; optical detectors; photoresistors; photodiodes; phototransistors; and/or quantum dot photoconductors/photodiodes. In other embodiments, step 620 may be performed by one or more of detectors 508 and 520 (FIG. 5).

Flow chart 600 proceeds from step 620 to step 624 where a first beam of a second light is emitted at the flow of fluid. In embodiments, the second light is different from the first light. For example, the second light may be of a different wavelength, frequency, and/or intensity. In one embodiment, the second light is of a different wavelength, e.g., longer or shorter, than the first light.

The light source used to emit the first beam of the second light at step 624 may be of any suitable type. In some embodiments, the light source is one or more of the light sources 404 and 416. The light sources that may be used include, electroluminescent sources such as (light emitting diodes (LEDs), electroluminescent sheets, electroluminescent wires, field-induced polymer electroluminescent (FIPEL)); electron-stimulated sources; incandescent sources; and/or gas discharge sources. Accordingly, in embodiments, the first light source may be one or more of LED's 504 and 516 (FIG. 5). In some embodiments, a light source to generate the first beam of the second light is activated at step 624, and the first beam of the second light is directed to the flow of fluid using light transmission methods/devices, some non-limiting examples including, fiber optics, reflectors (e.g., mirrors), or lenses.

At step 628, a first amount of the second light is detected. In embodiments, the first amount of the second light may be an amount of light that is transmitted through the flow of fluid, while in other embodiments the first amount of the second light may be an amount of the second light that is reflected by the flow of fluid. In yet other embodiments, step 628 may involve detecting both the amount of the second light that is transmitted through the flow of fluid, and the amount of the second light that is reflected by the flow of fluid.

Simlilar to step 620, step 628 may be performed by one or more light detectors. In embodiments, step 628 may be performed using one or more of detectors 408 and 420. Some examples of detectors that may be used in performing step 628 include, without limitation, active pixel sensors; charged-coupled devices; optical detectors; photoresistors; photodiodes; phototransistors; and/or quantum dot photoconductors/photodiodes. In other embodiments, step 628 may be performed by one or more of detectors 508 and 520.

At step 632, a baseline ratio is determined. In embodiments, the baseline ratio is a ratio of the first amount of the first light detected at step 620 to the first amount of the second light detected at step 628. In embodiments, the baseline ratio may be a ratio of the amount of the first light that is transmitted through the flow of fluid to the amount of the second light that is transmitted through the fluid flow. In other embodiments, the baseline ratio may be a ratio of the amount of the first light that is reflected by the flow of fluid to the amount of the second light that is reflected by the flow of fluid.

Step 632 may be performed by one or more microprocessors executing microprocessor executable instructions. In embodiments, the microprocessor may be part of a controller. As can be appreciated, in embodiments, signals from the detectors (detecting the amount of light at steps 620 and 628) may be used in determining the baseline ratio.

After step 632, a second beam of the first light is emitted at the flow of fluid. Step 636 may be performed by activating a light source, which is positioned so that when the second beam of the first light is emitted it is directed at the flow of fluid, which may be flowing through a conduit, such as tubing. In other embodiments, step 636 may involve activating a light source, and directing the second beam of the first light so that it is directed at the flow of fluid. As noted above with respect to step 616, the light source used to emit beams of the first light, at steps 616 and 636, may be of any suitable type.

Following step 636, a second amount of the first light is detected at step 640. The second amount of the first light may be from the second beam of the first light. In embodiments, the second amount of the first light may be an amount of the first light that is transmitted through the flow of fluid, while in other embodiments the second amount of the first light may be an amount of the first light that is reflected by the flow of fluid. In yet other embodiments, step 640 may involve detecting both the amount of the first light that is transmitted through the flow of fluid, and the amount of the second beam of first light that is reflected by the flow of fluid. Step 640 may be performed by one or more light detectors, similar to the detectors described above with respect to step 620.

Flow chart 600 proceeds from step 640 to step 644 where a second beam of the second light is emitted at the flow of fluid. As noted above, in embodiments, the second light is different from the first light, non-limiting examples of differences include, different wavelengths, frequency, and/or intensity. The light source used to emit the second beam of the second light at step 644 may be of any suitable type, as noted above with respect to step 624.

At step 648, a second amount of the second light is detected. The second amount of the second light may be from the second beam of the second light. The second amount of the second light may be an amount of light that is transmitted through the flow of fluid, while in other embodiments the second amount of the second light may be an amount of the second light that is reflected by the flow of fluid. In yet other embodiments, step 648 may involve detecting both the amount of the second light that is transmitted through the flow of fluid, and the amount of the second light that is reflected by the flow of fluid. Step 648 may be performed by one or more light detectors.

At step 652, a current ratio is determined. In embodiments, the current ratio is a ratio of the second amount of the first light detected at step 640 to the second amount of the second light detected at step 648. In embodiments, the current ratio may be a ratio of the amount of the first light (from the second beam) that is transmitted through the flow of fluid to the amount of the second light (from the second beam) that is transmitted through the flow of fluid. In other embodiments, the current ratio may be a ratio of the amount of the first light that is reflected by the flow of fluid to the amount of the second light that is reflected by the flow of fluid.

Step 652 may be performed by one or more microprocessors executing microprocessor executable instructions. In embodiments, the microprocessor may be part of a controller. As can be appreciated, in embodiments, signals from the detectors (detecting the amount of light at steps 640 and 648) may be used in determining the current ratio.

At decision 656, a determination is made whether the difference (in some embodiments the absolute value of the difference) between the current ratio and baseline ratio meets or exceeds a predetermined threshold. In embodiments, the threshold may be about ten percent (10%); about twenty percent (20%); about thirty percent (30%); about forty percent (40%); about fifty percent (50%); or even about sixty percent (60%). In some embodiments, the threshold may indicate how much less the current reading is than the previous reading. For example, if the ratios utilize a ratio of the first light transmitted to the second light transmitted through the flow of fluid, the threshold may be with respect to how much less the current ratio is than the baseline ratio. In those embodiments where the ratios utilize a ratio of the first light reflected to the second light reflected by the flow of fluid, the threshold may be with respect to how much more the current ratio is than the baseline ratio. If at decision 656 a determination is made that the difference does not meet the predetermined threshold, flow chart proceeds back to step 636, after which steps 636 through 652 are repeated.

When the composite fluid is blood, differences between the baseline ratio and current ratio may indicate that a different component is present in the flow of fluid. In one specific embodiment, the flow of fluid may comprise platelets and plasma. In these embodiments, the baseline ratio may be determined when platelets and plasma are in the flow of fluid. The current ratio may be continuously determined (e.g., step 636 through step 652) until a determination is made that the current ratio significantly varies (beyond a predetermined threshold) from the baseline ratio. This indicates, in embodiments, the presence of another component, such as white blood cells.

If at decision 656, a determination is made that the difference between the current ratio and baseline ratio meets or exceeds a predetermined threshold, flow chart 600 passes to step 660 where the first valve is closed. At optional step 664, a second valve may be opened to direct the flow of fluid into another container. Flow chart 600 ends at 668.

FIG. 7 illustrates a flow chart 700 that will be described, in parts, (see FIGS. 7A-7E) as illustrated in FIG. 7. In embodiments, flow chart 700 describes a process of separating a composite liquid into components and determining the presence of various components in a flow of fluid. The process of flow chart 700 may be performed in some embodiments by a separation device designed for separating composite fluids into components. For example, in embodiments, flow chart 700 may be performed by apparatus 60 (FIG. 2), which separates a plurality of discrete volumes of composite liquids into components. For purposes of simplicity, some of the steps of the process of flow chart 700 will be described below as being performed by a sensor such as sensor 500 (FIG. 5) and or apparatus 60 (FIG. 2) in separating whole blood into components. This is merely for purposes of illustration, and the process of flow chart 700 is not limited to being performed by any specific machine, apparatus, devices, or parts, or for separating any specific composite liquid.

Referring now to FIG. 7A, flow chart 700 starts at 702. In embodiments, other steps not shown in flow chart 700 may be performed after 702 but before step 704. In one embodiment, apparatus 60 may have been operated to separate one or more discrete volumes of blood into components. In other embodiments, the sensor 500 (FIG. 5) may be strobbed so that the blue LED 504 is lit and then both detectors 508 and 520 may be read. The blue LED 504 may then be turned off and the detectors 508 and 520 may be read again. Then the red LED 516 may be lit and again the detectors 508 and 520 read again. The red LED 516 may then be turned off and the detectors 508 and 520 may be read.

In embodiments, the detector (508 and 520) readings taken when the LED's 504 and 516 are dark are subtracted from the detector (508 and 520) readings when the LED's 504 and 516 were lit to cancel out the effects of background light. In embodiments, the detectors may be read with a 12-bit A/D converter.

As noted above with respect to FIG. 5, in some embodiments, the detectors (508 and 520) may be associated with one or more amplifiers that may be adjustable, such as with an 8-bit digital potentiometer. In embodiments, the steps after step 702 but before step 704 may include rotating a rotor (e.g., rotor 64) at a predetermined speed. Once the rotor has reached a hard spin speed and has idled there for some time (e.g., 1 minute), the gains of the detectors (508 and 520) may be adjusted so that the blue transmitted signal from the detector 508 is as close to 2000 as possible, (out of the 4095 available from a 12-bit A/D) and the red transmitted signal from the detector 520 is as close to 1750 as possible. After the hard spin time, the rotor speed may be reduced and a hydraulic pump (e.g., hydraulic pumping station 106) may be started to begin the expression of the blood components separated by apparatus 60.

The steps above may then be followed by the strobbing of the sensor 500 (e.g., activating and deactivating LED's 504 and 516, and reading detectors 508 and 520), as indicated above. In embodiments, the LED's 504 and 516 are strobbed as fast as possible, as long as there is at least 40 µs between the time the state of the LED changes and the time a detector is read.

In embodiments, some steps of flow chart 700 (see description below) involve the use of circular memory buffers for saving readings from the detectors. For example, when each detector (508 and 520) is read, the reading (adjusted for background light) may be compared to the previous reading. If the absolute value of the difference is less than 150, the reading may be stored into a buffer (in a memory, e.g., memory 86 (FIG. 8)) that holds the last 50 stored values. If the absolute value of the difference between the current reading and the previous reading is greater than 150, the current reading may be discarded and the value stored as a duplicate of the last stored value. Note that the comparison may be made with the last value read, not the last value stored. In embodiments, these will be the same, except for the reading just after a case where the difference was greater than 150. This process works to remove the effects of the strobe light used to watch the process.

The stored detector readings may then be averaged with a sliding window scheme where the current stored value is averaged with the previous three. The sliding window may be an average of 4 values. These averaged values may be stored in another buffer containing the most recent 50 averaged values.

Embodiments may utilize circular buffers holding the most recent 50 averaged values. In one embodiment, there are six (6) buffers. For example, the buffers may include:
1. Blue transmitted
2. Blue reflected
3. Red transmitted
4. Red reflected
5. The ratio of Blue transmitted to Red transmitted (Bt / Rt)
6. Bucket sensor

In some embodiments, steps between step 702 and step 704 may further include running a centrifuge of apparatus 60 (FIG. 2). In embodiments, the centrifuge 60 is run to 1500 RPM, when the centrifuge 60 is first started. During the initial acceleration, a plasma valve may be open. When the plasma valve is open, the centrifuge speed may be ramped up to 3200 RPM. After the hard spin time is complete, the centrifuge speed may be ramped down to 1800 RPM. In embodiments, the ramp rate may be gradual. When the centrifuge speed reaches 1800 RPM, the plasma valve may be closed and a platelet valve may be opened. The hydraulic rate, e.g., rate that the hydraulic pumping station 106 is pumping hydraulic fluid, may be set to a predetermined rate. In embodiments, the rate may be less than six hundred (600) ml/min, less than five hundred fifty (550) ml/min, less than five hundred (500) ml/min, less than four hundred fifty (450) ml/min, or even less than four hundred (400) ml/min. In other embodiments, the rate may be greater than two hundred (200) ml/min, greater than two hundred fifty (250) ml/min, greater than three hundred (300) ml/min, greater than three hundred fifty (350) ml/min, or even greater than four hundred (400) ml/min. In one embodiment, the rate is about four hundred fifty (450) ml/min. With the hydraulics running for bulk expression, the memory buffers may be filled for about two seconds.

Referring back to flow chart 700 and FIG. 7A, the tube that is in the pathway through the sensor (e.g., sensor 500) is first empty. Step 704 involves detecting the values of the first detector 508 and the second detector 520. The detector 508 and 520 are read to determine a current reading of blue light that is transmitted through the tube, which as noted above may be empty. Step 704 also involves reading detector 508 and 520 to determine a current reading of red light that is transmitted through the tube. The amount of blue light and red light transmitted is compared to the value read a little less than a second previously. It is noted that in some embodiments, the detectors may be used to determine the amount of reflected blue and or red light.

At decision 706, a determination is made if either the blue and/or red current readings are higher (or meet a threshold difference) than the readings taken at least a second before. In some embodiments, step 706 may determine if the either the blue and/or red current readings are about five percent (5%) higher, about seven percent (7%) higher, about ten percent (10%) higher, or even about fifteen percent (15%) higher than the readings taken at least a second before. If the readings are not higher, flow 700 loops back to step 704 where the detector readings are made again.

If at decision 706, a determination is made that current reading of the blue and/or red transmitted light is higher than the readings taken at least a second before, flow passes to step 710, where the hydraulic pumping station may be stopped; the platelet valve may be closed; and a plasma valve may be opened, after which the hydraulics station may be started again at its previous rate or a different rate. In embodiments, the rate may be less than six hundred (600) ml/min, less than five hundred fifty (550) ml/min, less than five hundred (500) ml/min, less than four hundred fifty (450) ml/min, or even less than four hundred (400) ml/min. In other embodiments, the rate may be greater than two hundred (200) ml/min, greater than two hundred fifty (250) ml/min, greater than three hundred (300) ml/min, greater than three hundred fifty (350) ml/min, or even greater than four hundred (400) ml/min. In one embodiment, the rate is restarted at about four hundred fifty (450) ml/min. In embodiments, restarting of the hydraulic pumping station results in a component of the whole blood, e.g., plasma being pumped through the tubing positioned in the pathway of sensor 500.

After step 710, flow chart 700 passes to step 712 where the system waits for a predetermined amount of time. In embodiments, step 712 is performed to allow any foam or air to pass through the tubing before sensor 500 may be operated to once again detect light transmitted and reflected through the flow of fluid in the tubing.

In embodiments, the amount of time the system waits during step 712, may be greater than one (1) second; greater than about two (2) seconds; greater than about three (3) seconds; greater than about four (4) seconds; or greater than about five (5) seconds. In other embodiments, the amount of time the system waits during step 712, may be less than about thirty (30) seconds; less than about twenty five (25) seconds; less than about twenty (20) seconds; or less than about fifteen (15) seconds. In one embodiment, the amount of time the system waits during step 712 may be about ten (10) seconds.

After step 712, a minimum and a maximum value of a bucket sensor are set at step 714. The bucket sensor may be read using an A/D converter. In one embodiment, the minimum bucket sensor may be set at about four thousand (4000) and the maximum may be set at about zero (0). As can be appreciated, in other embodiments, the minimum and maximum values may be set at other appropriate values depending on the type of sensor, its output, or the type of A/D converter used to read the sensor.

At decision 716, each bucket sensor reading is compared to the minimum value set at step 714. If the reading is less than the minimum, the minimum value is then replaced with the reading at step 718. Flow chart 700 then passes back to decision 716.

If at decision 716, the reading of each bucket sensor is greater than the minimum, flow chart 700 passes to decision 720 where a determination is made whether the current read value of the bucket sensor is greater than the minimum by a predetermined amount. The predetermined amount may be any appropriate amount. If at decision 716 a determination is made that the current read value is less than the minimum, flow chart 700 passes to step 718 where the current value is stored as the new minimum. Flow chart 700 then passes back to step 716.

If the reading is greater than the minimum by the predetermined amount, flow chart 700 passes to step 722 (FIG. 7B), where each bucket sensor reading is compared to the maximum value to determine if the reading is greater than the maximum value. If the reading is greater than the maximum value set at step 714, then flow passes to step 724 where the maximum value is replaced with the current reading and flow passes back to decision 722.

If at decision 722 it is determined that the reading is less than the maximum, flow chart 700 passes to decision 726 where a determination is made whether the current reading is less than some predetermined fraction of the maximum value. In some embodiments, the predetermined fraction may be about ten percent (10%); about twenty percent (20%); about thirty percent (30%); about forty percent (40%) about fifty percent (50%); or even about sixty percent (60%). If at decision 726, a determination is made that the reading is not less than the predetermined fraction of the maximum value flow chart 700 passes back to decision 722.

If at decision 726, a determination is made that the reading is less than the predetermined fraction of the maximum value, flow chart 700 passes to step 728 where the system waits a predetermined period of time. In embodiments, the predetermined period of time may be less than about one (1) second, less than about two (2) seconds, less than about three (3) seconds, less than about four (4) seconds, less than about five (5) seconds, or even less than about six (6) seconds. Step 728 is designed in some embodiments to allow a volume of the plasma to be transferred during the waiting.

After the predetermined period of time passes, the hydraulic pumping station is stopped at step 730. Also, at step 730, the plasma valve may be closed, the platelet valve may be opened, and the hydraulic pumping station may be restarted. In embodiments, the hydraulic pumping station is started at a predetermined rate. The rate may be, in some embodiments, less than two hundred (200) ml/min, less than one hundred fifty (150) ml/min, less than one hundred (100) ml/min, less than fifty (50) ml/min, or even less than twenty five (25) ml/min. In other embodiments, the rate may be, greater than ten (10) ml/min, greater than twenty (20) ml/min, greater than thirty (30) ml/min, greater than forty (40) ml/min, or even greater than fifty (50) ml/min. Plasma may begin flowing into a platelet bag when the hydraulic pumping station is restarted.

After step 730, plasma may be in the tubing, and may be directed to the platelet bag before the platelets. Flow chart 700 moves to step 732 (FIG. 7C) where the pumping station is run for a predetermined period of time. In embodiments, the time is selected to move a buffy coat (containing platelets and white blood cells) of the separated blood close to a funnel output port of the bucket. In embodiments, the time period may be less than sixty (60) seconds, less than fifty (50) seconds, less than forty (40) seconds, less than thirty (30) seconds, or even less than twenty (20) seconds. In other embodiments, the period of time may be, greater than five (5) seconds, greater than ten (10) seconds, greater than fifteen (15) seconds, greater than twenty (20) seconds, or even greater than twenty five (25) seconds. In one embodiment, the time period is about twenty three (23) seconds.

After step 732, flow chart 700 moves to step 734 where the first detector 508 and the second detector 520 are read. In embodiments, the detectors 508 and 520 are read to determine an amount of blue transmitted light and red transmitted light that is transmitted through the flow. In other embodiments, the detectors may be read to determine an amount of blue reflected light and red reflected light that is reflected by the flow of fluid in the tubing.

At decision 736 a comparison is made between a current ratio of the blue detected light to the red detected light (e.g., blue transmitted/red transmitted or blue reflected/red reflected) and the ratio determined earlier, e.g., less than one (1) second earlier, less than two (2) seconds earlier, less than three (3) seconds earlier, less than four (4) seconds earlier, or even less than five (5) seconds earlier. A determination is made, at decision 736, whether the current ratio differs from the previous ratio (determined earlier) by a threshold amount. In embodiments, the threshold may be about ten percent (10%); about twenty percent (20%); about thirty percent (30%); about forty percent (40%) about fifty percent (50%); or even about sixty percent (60%). In some embodiments, the threshold may indicate how much less the current reading is than the previous reading. For example, if the ratios utilize a ratio of the blue light transmitted to the red light transmitted through the flow of fluid, the threshold may be with respect to how much less the current ratio is than the previous ratio. In those embodiments where the ratios utilize a ratio of the blue light reflected to the red light reflected by the flow of fluid, the threshold may be with respect to how much more the current ratio is than the previous ratio. In embodiments, the threshold is ten percent (10%), and the ratios are blue light transmitted through the flow of fluid to red light transmitted through the flow of fluid.

If at decision 736 the ratio does not differ by the threshold amount, flow chart 700 passes to step 738 where the system waits a period of time. The predetermined period of time may be less than about one (1) second, less than about two (2) seconds, less than about three (3) seconds, less than about four (4) seconds, less than about five (5) seconds, or even less than about six (6) seconds. Flow chart 700 then passes back to step 734.

If at decision 736 the ratio does differ by the threshold amount, flow chart 700 passes to step 740. In embodiments, when the ratio does differ by the threshold amount, it means that platelets are in the tube and going to the platelet bag.

At step 740, the current value of red light is stored. In embodiments, this may involve storing (in a buffer) the value from detector 520, detecting the current red light transmitted through the flow of fluid. In other embodiments, step 740 may involve reading and storing an amount of the red light that is reflected by the flow of fluid.

Also at step 740, the current ratio of blue light detected to red light detected is determined and stored (in a buffer). In embodiments, this may involve determining and storing the blue light transmitted/red light transmitted. In other embodiments, this may involve determining and storing the blue light reflected/red light reflected. In embodiments, the red light detected and the ratio of blue light to red light are stored as baseline values.

Flow chart 700 proceeds to step 742 where red light is detected. In embodiments, it is the amount of red light transmitted through the flow of fluid and in other embodiments it is the amount of red light reflected by the flow of fluid. Following step 742, at decision 744, a determination is made whether the current red light value detected differs from the baseline value by more than a threshold amount. In embodiments, the red light detected value is an amount of red light transmitted through the flow, and the threshold amount is less than a fraction of the baseline, such as, for example, less than about eighty percent (80%) of the baseline, less than about eighty five percent (85%) of the baseline, less than about ninety percent (90%) of the baseline, or even less than about ninety five percent (95%) of the baseline. In one embodiment, the threshold is less than about ninety two (92%) of the baseline.

If at decision 744, a determination is made that the current red light value detected does not differ from the baseline by at least the threshold amount, flow chart 700 passes back to step 742. If at decision 744, a determination is made that the current red light value detected does differ from the baseline by the threshold value, flow chart 700 passes to step 746, where a timer is started. The timer may be set to any suitable amount of time for platelets to flow into the platelet storage bag. In embodiments, the timer may be set for about one (1) second, about two (2) seconds, about three (3) seconds, about four (4) seconds, about five (5) seconds, or even about six (6) seconds. In one embodiment, the timer is set to about 1.5 seconds.

Flow chart 700 then passes to decisions 748, 750, 752, and 754 (FIG. 7D). It is noted that the decisions 748, 750, 752, and 754 do not have to necessarily occur in the order shown in FIG. 7D. The order of these decisions is provided merely for illustrative purposes. As is described below, at any decision 748, 750, 752, and 754 a determination may result in flow chart 700 passing to step 756. Otherwise, the decisions may result in looping back through the decisions 748, 750, 752, and 754. Therefore, in other embodiments, flow chart 700 may have decisions 748, 750, 752, and 754 in any order including an order that is different than the order shown in FIG. 7D with the possibility of any decision resulting in passing to the next step or in looping back through the decisions.

At decision 748, a determination is made whether the timer set at step 746 has timed out. If the timer has timed out, flow passes to step 756 where the hydraulic pumping station is stopped. Also, at step 756, the platelet valve is closed, the waste valve is opened, and then the hydraulic pumping station is started again. In embodiments, the hydraulic pumping station is restarted at a predetermined rate. The rate may be, in some embodiments, less than two hundred (200) ml/min, less than one hundred fifty (150) ml/min, less than one hundred (100) ml/min, less than fifty (50) ml/min, or even less than twenty five (25) ml/min. In other embodiments, the rate may be, greater than ten (10) ml/min, greater than twenty (20) ml/min, greater than thirty (30) ml/min, greater than forty (40) ml/min, or even greater than fifty (50) ml/min. White blood cells in the flow may begin flowing into a waste bag when the hydraulic pumping station is restarted.

If at decision 748, a determination is made that the timer has not timed out, flow chart 700 passes to decision 750, where a determination is made whether a current red light detection value differs from the baseline value by a threshold amount, or is at some threshold value. In embodiments, the red light detected as part of decision 750 is the amount of red light transmitted through the flow of fluid, while in other embodiments it is the amount of red light reflected by the flow of fluid. Therefore in embodiments, the red light detected is an amount of red light transmitted through the flow, and the threshold amount is less than a fraction of the baseline, such as, for example less than about thirty percent (30%) of the baseline, less than about twenty five percent (25%) of the baseline, less than about twenty percent (20%) of the baseline, less than about fifteen percent (15%) of the baseline, less than about ten percent (10%) of the baseline, or even less than about five percent (5%) of the baseline. In one embodiment, the threshold is less than about three percent (3%) of the baseline.

If at decision 750 a determination is made that the current red light detection value does differ from the previous value by the threshold amount, flow chart 700 passes to step 756. If at decision 750 a determination is made that the current red light detection value does not differ from the baseline by the threshold amount, flow chart 700 passes to decision 752.

At decision 752 a determination is made whether a ratio of detected blue light to detected red light differs from the baseline value by a threshold amount. In embodiments, decision 752 may involve determining the ratio of blue transmitted light to red transmitted light. In embodiments, the threshold amount is a fraction of the baseline, such as, for example, less than about thirty percent (30%) of the baseline, less than about twenty five percent (25%) of the baseline, less than about twenty percent (20%) of the baseline, less than about fifteen percent (15%) of the baseline, less than about ten percent (10%) of the baseline, or even less than about five percent (5%) of the baseline. In one embodiment, the threshold is less than about two percent (2%) of the baseline. In other embodiments, the ratio determined at decision 752 may be the amount of blue light reflected to the amount of red light reflected by the flow of fluid. In these embodiments, the threshold may be greater than some fraction of the baseline.

If at decision 752 a determination is made that the current ratio of blue light to red light detected does differ from the baseline by the threshold amount, flow chart 700 passes to step 756. If at decision 752 a determination is made that the current ratio of blue light to red light detected does not differ from the baseline by the threshold amount, flow chart 700 passes to decision 754.

At decision 754 a determination is made whether a sum of all of the blue light and red light detected, whether transmitted or reflected, is less than a threshold amount. The amount may be expressed in any suitable units. In embodiments where the detectors are connected to a 12-bit A/D converter, the threshold amount may be expressed as counts. In these embodiments the threshold may be, less than one hundred (100), less than ninety (90), less than eighty (80), less than seventy (70), less than sixty (60), less than fifty (50), less than forty (40), less than thirty (30) or even less than twenty (20) counts.

If at decision 754 a determination is made that the sum of all of the blue light and all of the red light detected is less than the threshold amount, flow chart 700 passes to step 756. If at decision 754 a determination is made that the sum of all of the blue light detect and all of the red light detected is not less than the threshold amount, flow chart 700 loops to decision 748.

After step 756, flow chart 700 passes to decision 758 (Fig. 7E) where a determination is made whether the sum of all of the blue light and the red light, whether transmitted or reflected drop below a threshold amount. If so, flow passes to step 760. At step 760, a timer is set, and the flow is stopped.

If at decision 758 a determination is made that the sum of the blue light and red light is not less than the threshold amount, flow chart 700 passes to decision 764. At decision 764 a determination is made whether a predetermined period of time has passed. In embodiments, the predetermined period of time may be less than about forty (40) seconds, less than about thirty five (35) seconds, less than about thirty (30) seconds, less than about twenty five (25) seconds, less than about twenty (20) seconds, or even less than about fifteen (15) seconds. In some embodiment, the predetermined period of time is about 15 seconds.

If at decision 764, a determination is made that the predetermined period of time has passed, flow chart 700 proceeds to step 760. If at decision 764, a determination is made that the predetermined period of time has not passed, flow chart 700 loops back to decision 758.

After step 760, flow chart 700 progresses to step 762, where another volume of whole blood may be processed according to flow chart 700. In embodiments, several discrete volumes of whole blood may be separated, and flow chart 700 may be performed on each volume of whole blood to remove the separate components, once separated. Flow chart 700 ends at 766.

Although flow charts 600 (FIG. 6) and 700 (FIG. 7) have been described with steps listed in a particular order, the present invention is not limited thereto. In other embodiments, steps may be performed in different order, in parallel, or any different number of times, e.g., before and after another step. Also, as indicated above, flow charts 600 and 700 include some optional steps. However, those steps above that are not indicated as optional should not be considered as essential to the invention, but may be performed in some embodiments of the present invention and not in others.

FIG. 8 illustrates example components of a basic computer system 800 upon which embodiments of the present invention may be implemented. For example, controller 157 may incorporate features of the basic computer system 800 shown in FIG. 8. For example, in one embodiment controller 157 may include at least memory 816 and processor 812 described below.

Computer system 800 includes output device(s) 804, and input device(s) 808. Output device(s) 804 may include, among other things, one or more displays, including CRT, LCD, and/or plasma displays. Output device(s) 804 may also include printers, speakers etc. Input device(s) 808 may include a keyboard, touch input devices, a mouse, voice input device, scanners, etc.

Basic computer system 800 may also include a processing unit 812 and memory 816, according to embodiments of the present invention. The processing unit 812 may be a general purpose processor operable to execute processor executable instructions stored in memory 816. Processing unit 812 may include a single processor or multiple processors, according to embodiments. Further, in embodiments, each processor may be a single core or a multi-core processor, having one or more cores to read and execute separate instructions. The processors may include general purpose processors, application specific integrated circuits (ASICs), field programmable gate arrays (FPGAs), and other integrated circuits.

The memory 816 may include any tangible storage medium for short-term or long-term storage of data and/or processor executable instructions. The memory 816 may include, for example, Random Access Memory (RAM), Read-Only Memory (ROM), or Electrically Erasable Programmable Read-Only Memory (EEPROM). Other storage media may include, for example, CD-ROM, tape, digital versatile disks (DVD) or other optical storage, tape, magnetic disk storage, magnetic tape, other magnetic storage devices, etc.

Storage 828 may be any long-term data storage device or component. Storage 828 may include one or more of the devices described above with respect to memory 816. Storage 828 may be permanent or removable.

Computer system 800 also includes communication devices 836. Devices 836 allow system 800 to communicate over networks, e.g., wide area networks, local area networks, storage area networks, etc., and may include a number of devices such as modems, hubs, network interface cards, wireless network interface cards, routers, switches, bridges, gateways, wireless access points, etc.

The components of computer system 800 are shown in FIG. 8 as connected by system bus 840. It is noted, however, that in other embodiments, the components of system 800 may be connected using more than a single bus.

In embodiments, memory 816 may store information in buffers 820. For example, buffers 820 may store values such as amounts of light (e.g., blue light or red light) detected by detectors, ratios of detected light, or other data. Storage 828 may store more permanent data, such as predetermined periods of time 824 or thresholds 832 utilized in procedures, processes, or methods such as the processes illustrated in flow charts 600 (FIG. 6) and 700 (FIG. 7) and described above.

It will be apparent to those skilled in the art that various modifications and variations can be made to the methods and structure of the present invention without departing from its scope. Thus it should be understood that the invention is not be limited to the specific examples given. Rather, the invention is intended to cover modifications and variations within the scope of the following claims and their equivalents. The steps, features, structures, and/or media are disclosed as illustrative embodiments for implementation of the claims and are not intended to limit the claims.

While example embodiments and applications of the present invention have been illustrated and described, it is to be understood that the invention is not limited to the precise configuration and resources described above. Various modifications, changes, and variations apparent to those skilled in the art may be made in the arrangement, operation, and details of the method(s) and apparatus of the present invention disclosed herein without departing from the scope of the claimed invention.

## Claims

1. A method of controlling flow of components separated from whole blood, the method comprising:
opening (612) a first valve (128) to allow a flow of platelets separated from the whole blood into a first container (16);
emitting (616) a beam of a first visible light at the flow of platelets;
detecting (620), with a first sensor, a first amount of the first visible light;
emitting (624) a beam of a second visible light at the flow of platelets;
detecting (628), with a second sensor, a first amount of the second visible light;
determining (632), by at least one processor (812), a baseline ratio, wherein the baseline ratio comprises a ratio of the first amount of the first visible light to the first amount of the second visible light;
after a period of time:
emitting (636) a second beam of the first visible light at the flow of platelets;
detecting (640), with the first sensor, a second amount of the first visible light;
emitting (644) a second beam of the second visible light at the flow of platelets;
detecting (648), with the second sensor, a second amount of the second visible light;
determining (652), by the at least one processor (812), a current ratio, wherein the current ratio comprises a ratio of the second amount of the first visible light to the second amount of the second visible light;
determining (656), by the at least one processor (812), whether a difference between the current ratio and the baseline ratio meets a predetermined threshold indicating the presence of white blood cells; and
closing (660) the first valve (128) to stop the flow of platelets into the first container (16) if the difference between the current ratio and the baseline ratio meets the predetermined threshold.

2. The method of claim 1, wherein the first visible light has a smaller wavelength than the second visible light.

3. The method of claim 1 or claim 2, wherein the first visible light comprises blue visible light.

4. The method of any one of claims 1-3, wherein the second visible light comprises red visible light.

5. The method of any one of claims 1-4, further comprising: after the determination that the difference between the current ratio and the baseline ratio meets the predetermined threshold, opening a second valve to allow a flow of white blood cells into a second container.

6. The method of any one of claims 1-5, wherein:
the first amount of the first visible light comprises a first amount of the first visible light that is transmitted through the flow of platelets;
the first amount of the second visible light comprises a first amount of the second visible light that is transmitted through the flow of platelets;
the second amount of the first visible light comprises a second amount of the first visible light that is transmitted through the flow of platelets; and
the second amount of the second visible light comprises a second amount of the second visible light that is transmitted through the flow of platelets.

7. The method of any one of claims 1-5, wherein:
the first amount of the first visible light comprises a first amount of the first visible light that is reflected by the flow of platelets;
the first amount of the second visible light comprises a first amount of the second visible light that is reflected by the flow of platelets;
the second amount of the first visible light comprises a second amount of the first visible light that is reflected by the flow of platelets; and
the second amount of the second visible light comprises a second amount of the second visible light that is reflected by the flow of platelets.

8. The method of any one of claims 1-7, further comprising: before opening the first valve, separating the whole blood into components.

9. The method of claim 8, wherein the separating comprises centrifuging the whole blood.

10. The method of claim 9, wherein the separating comprises centrifuging a plurality of bags of whole blood.

11. The method of any one of claims 1-10, wherein the difference is an absolute value of the difference.

12. An apparatus (60) for separating components from whole blood, the apparatus comprising
a rotor (64) having at least one separation cell (78), wherein the at least one separation cell is configured to receive a separation bag (12) containing whole blood
at least one collection cavity (154),
wherein the at least one collection cavity is configured to receive at least a first collection bag (14) and a second collection bag (16);
a first sensor (408, 508) for detecting visible light;
a second sensor (420, 520) for detecting visible light
a first visible light source (404, 504);
a second visible light source (416, 516);
a first valve (128) for controlling flow into the first collection bag a second valve (132) for controlling flow into the second collection bag; and
at least one processor (812) for executing computer readable instructions that when executed by the at least one processor performs the steps of any one of claims 1-11.

13. The apparatus of claim 12, wherein the first detector is positioned to detect first light that is transmitted through the flow, and to detect second light that is reflected by the flow of the first component.

14. The apparatus of any one of claim 12 or claim 13, wherein the second detector is positioned to detect second light that is transmitted through the flow, and detect first light that is reflected by the flow of the first component.

15. The apparatus of any one of claims 12-14, further comprising a plurality of separation cells and a plurality of collection cavities.

## Patentansprüche

1. Verfahren zum Steuern des Stroms von aus Vollblut getrennten Komponenten, wobei das Verfahren umfasst:
Öffnen (612) eines ersten Ventils (128), um einen Strom von aus dem Vollblut getrennten Blutplättchen in einen ersten Behälter (16) zu ermöglichen;
Emittieren (616) eines Strahls eines ersten sichtbaren Lichts auf den Strom von Blutplättchen;
Erkennen (620), mit einem ersten Sensor, einer ersten Menge des ersten sichtbaren Lichts;
Emittieren (624) eines Strahls eines zweiten sichtbaren Lichts auf den Strom von Blutplättchen;
Erkennen (628), mit einem zweiten Sensor, einer ersten Menge des zweiten sichtbaren Lichts;
Bestimmen (632), durch wenigstens einen Prozessor (812), eines Basislinienverhältnisses, wobei das Basislinienverhältnis ein Verhältnis der ersten Menge des ersten sichtbaren Lichts zu der ersten Menge des zweiten sichtbaren Lichts umfasst;
nach einer Zeitraum:
Emittieren (636) eines zweiten Strahls des ersten sichtbaren Lichts auf den Strom von Blutplättchen;
Erkennen (640), mit dem ersten Sensor, einer zweiten Menge des ersten sichtbaren Lichts;
Emittieren (644) eines zweiten Strahls des zweiten sichtbaren Lichts auf den Strom von Blutplättchen;
Erkennen (648), mit dem zweiten Sensor, einer zweiten Menge des zweiten sichtbaren Lichts;
Bestimmen (652), durch den wenigstens einen Prozessor (812), eines aktuellen Verhältnisses, wobei das aktuelle Verhältnis ein Verhältnis der zweiten Menge des ersten sichtbaren Lichts zu der zweiten Menge des zweiten sichtbaren Lichts umfasst;
Bestimmen (656), durch den wenigstens einen Prozessor (812), ob eine Differenz zwischen dem aktuellen Verhältnis und der Basislinie eine vorbestimmte Schwelle erfüllt, die das Vorhandensein von weißen Blutkörperchen anzeigt; und
Schließen (660) des ersten Ventils (128), um den Strom von Blutplättchen in den ersten Behälter (16) zu stoppen, wenn die Differenz zwischen dem aktuellen Verhältnis und dem Basislinienverhältnis die vorbestimmte Schwelle erfüllt.

2. Verfahren nach Anspruch 1, wobei das erste sichtbare Licht eine kleinere Wellenlänge aufweist als das zweite sichtbare Licht.

3. Verfahren nach Anspruch 1 oder 2, wobei das erste sichtbare Licht blaues sichtbares Licht umfasst.

4. Verfahren nach einem der Ansprüche 1-3, wobei das zweite sichtbare Licht rotes sichtbares Licht umfasst.

5. Verfahren nach einem der Ansprüche 1-4, das ferner umfasst:
nach der Bestimmung, dass die Differenz zwischen dem aktuellen Verhältnis und dem Basislinienverhältnis die vorbestimmte Schwelle erfüllt, das Öffnen eines zweiten Ventils, um einen Strom von weißen Blutzellen in einen zweiten Behälter zu ermöglichen.

6. Verfahren nach einem der Ansprüche 1-5, wobei:
die erste Menge des ersten sichtbaren Lichts eine erste Menge des ersten sichtbaren Lichts umfasst, die durch den Strom von Blutplättchen übertragen wird;
die erste Menge des zweiten sichtbaren Lichts eine erste Menge des zweiten sichtbaren Lichts umfasst, die durch den Strom von Blutplättchen übertragen wird;
die zweite Menge des ersten sichtbaren Lichts eine zweite Menge des ersten sichtbaren Lichts umfasst, die durch den Strom von Blutplättchen übertragen wird; und
die zweite Menge des zweiten sichtbaren Lichts eine zweite Menge des zweiten sichtbaren Lichts umfasst, das durch den Strom von Blutplättchen übertragen wird.

7. Verfahren nach einem der Ansprüche 1-5, wobei:
die erste Menge des ersten sichtbaren Lichts eine erste Menge des ersten sichtbaren Lichts umfasst, die von dem Strom von Blutplättchen reflektiert wird;
die erste Menge des zweiten sichtbaren Lichts eine erste Menge des zweiten sichtbaren Lichts umfasst, die von dem Strom von Blutplättchen reflektiert wird;
die zweite Menge des ersten sichtbaren Lichts eine zweite Menge des ersten sichtbaren Lichts umfasst, die von dem Strom der Blutplättchen reflektiert wird; und
die zweite Menge des zweiten sichtbaren Lichts eine zweite Menge des zweiten sichtbaren Lichts umfasst, die von dem Strom von Blutplättchen reflektiert wird.

8. Verfahren nach einem der Ansprüche 1-7, ferner umfassend:
vor dem Öffnen des ersten Ventils, Trennen des Vollbluts in Komponenten.

9. Verfahren nach Anspruch 8, wobei die Trennung das Zentrifugieren des Vollbluts umfasst.

10. Verfahren nach Anspruch 9, wobei das Trennen das Zentrifugieren einer Mehrzahl von Beuteln mit Vollblut umfasst.

11. Verfahren nach einem der Ansprüche 1-10, wobei die Differenz ein absoluter Wert der Differenz ist.

12. Einrichtung (60) zum Trennen von Komponenten aus Vollblut, wobei die Einrichtung umfasst:
einen Rotor (64) mit wenigstens einer Trennzelle (78), wobei die wenigstens eine Trennzelle dazu konfiguriert ist, einen Trennbeutel (12) aufzunehmen, der Vollblut enthält,
wenigstens einen Sammelhohlraum (154), wobei der wenigstens eine Sammelhohlraum dazu konfiguriert ist, wenigstens einen ersten Sammelbeutel (14) und einen zweiten Sammelbeutel (16) aufzunehmen;
einen ersten Sensor (408, 508) zum Erkennen von sichtbarem Licht;
einen zweiten Sensor (420, 520) zum Erkennen von sichtbarem Licht;
eine erste Quelle für sichtbares Licht (404, 504);
eine zweite Quelle für sichtbares Licht (416, 516);
ein erstes Ventil (128) zum Steuern des Stroms in den ersten Sammelbeutel;
ein zweites Ventil (132) zum Steuern des Stroms in den zweiten Sammelbeutel; und
wenigstens einen Prozessor (812) zum Ausführen von computerlesbaren Anweisungen, die, wenn sie von dem wenigstens einen Prozessor ausgeführt werden, die Schritte nach einem der Ansprüche 1-11 durchführen.

13. Einrichtung nach Anspruch 12, wobei der erste Detektor positioniert ist, um erstes Licht zu erkennen, das durch den Strom übertragen wird, und zweites Licht zu erkennen, das von dem Strom der ersten Komponente reflektiert wird.

14. Einrichtung nach Anspruch 12 oder 13, wobei der zweite Detektor positioniert ist, um zweites Licht zu erkennen, das durch den Strom übertragen wird, und erstes Licht zu erkennen, das von dem Strom der ersten Komponente reflektiert wird.

15. Einrichtung nach einem der Ansprüche 12-14, ferner umfassend eine Mehrzahl von Trennzellen und eine Mehrzahl von Sammelhohlräumen.

## Revendications

1. Procédé de contrôle du flux de composants séparés du sang total, le procédé comprenant :
l'ouverture (612) d'une première vanne (128), pour permettre un flux de plaquettes séparées du sang total dans un premier récipient (16) ;
l'émission (616) d'un faisceau d'une première lumière visible au niveau du flux de plaquettes ;
la détection (620), avec un premier capteur, d'une première quantité de la première lumière visible ;
l'émission (624) d'un faisceau d'une deuxième lumière visible au niveau du flux de plaquettes ;
la détection (628), avec un deuxième capteur, d'une première quantité de la deuxième lumière visible ;
la détermination (632), par au moins un processeur (812), d'un rapport de base, dans lequel le rapport de base comprend un rapport de la première quantité de la première lumière visible par rapport à la première quantité de la deuxième lumière visible ;
après un certain temps :
l'émission (636) d'un deuxième faisceau de la première lumière visible au niveau du flux de plaquettes ;
la détection (640), avec le premier capteur, d'une deuxième quantité de la première lumière visible ;
l'émission (644) d'un deuxième faisceau de la deuxième lumière visible au niveau du flux de plaquettes ;
la détection (648), avec le deuxième capteur, d'une deuxième quantité de la deuxième lumière visible ;
la détermination (652), par l'au moins un processeur (812), d'un rapport actuel, dans lequel le rapport actuel comprend un rapport de la deuxième quantité de la première lumière visible par rapport à la deuxième quantité de la deuxième lumière visible ;
la détermination (656), par l'au moins un processeur (812), si une différence entre le rapport actuel et le rapport de base satisfait à un seuil prédéterminé indiquant la présence de globules blancs ; et
la fermeture (660) de la première vanne (128) pour arrêter le flux de plaquettes dans le premier récipient (16) si la différence entre le rapport actuel et le rapport de base atteint le seuil prédéterminé.

2. Procédé selon la revendication 1, dans lequel la première lumière visible a une longueur d'onde inférieure à celle de la deuxième lumière visible.

3. Procédé selon la revendication 1 ou la revendication 2, dans lequel la première lumière visible comprend de la lumière visible bleue.

4. Procédé selon l'une quelconque des revendications 1 à 3, dans lequel la deuxième lumière visible comprend de la lumière visible rouge.

5. Procédé selon l'une quelconque des revendications 1 à 4, comprenant en outre :
après la détermination, que la différence entre le rapport actuel et le rapport de base atteint le seuil prédéterminé, l'ouverture d'une deuxième vanne pour permettre un écoulement de globules blancs dans un deuxième récipient.

6. Procédé selon l'une quelconque des revendications 1 à 5, dans lequel :
la première quantité de la première lumière visible comprend une première quantité de la première lumière visible qui est transmise à travers le flux de plaquettes ;
la première quantité de la deuxième lumière visible comprend une première quantité de la deuxième lumière visible qui est transmise à travers le flux de plaquettes ;
la deuxième quantité de la première lumière visible comprend une deuxième quantité de la première lumière visible qui est transmise à travers le flux de plaquettes ; et
la deuxième quantité de la deuxième lumière visible comprend une deuxième quantité de la deuxième lumière visible qui est transmise à travers le flux de plaquettes.

7. Procédé selon l'une quelconque des revendications 1 à 5, dans lequel :
la première quantité de la première lumière visible comprend une première quantité de la première lumière visible qui est réfléchie par le flux de plaquettes ;
la première quantité de la deuxième lumière visible comprend une première quantité de la deuxième lumière visible qui est réfléchie par le flux de plaquettes ;
la deuxième quantité de la première lumière visible comprend une deuxième quantité de la première lumière visible qui est réfléchie par le flux de plaquettes ; et
la deuxième quantité de la deuxième lumière visible comprend une deuxième quantité de la deuxième lumière visible qui est réfléchie par le flux de plaquettes.

8. Procédé selon l'une quelconque des revendications 1 à 7, comprenant en outre :
avant l'ouverture de la première vanne, la séparation du sang total en composants.

9. Procédé selon la revendication 8, dans lequel la séparation comprend la centrifugation du sang total.

10. Procédé selon la revendication 9, dans lequel la séparation comprend la centrifugation d'une pluralité de poches de sang total.

11. Procédé selon l'une quelconque des revendications 1 à 10, dans lequel la différence est une valeur absolue de la différence.

12. Appareil (60) de séparation de composants du sang total, l'appareil comprenant
un rotor (64) ayant au moins une cellule de séparation (78), dans lequel l'au moins une cellule de séparation est configurée pour recevoir une poche de séparation (12) contenant du sang total ;
au moins une cavité de collecte (154), dans lequel l'au moins une cavité de collecte est configurée pour recevoir au moins un premier sac de collecte (14) et un deuxième sac de collecte (16) ;
un premier capteur (408, 508) pour détecter la lumière visible ;
un deuxième capteur (420, 520) pour détecter la lumière visible ;
une première source de lumière visible (404, 504) ;
une deuxième source de lumière visible (416, 516) ;
une première vanne (128) pour contrôler le flux dans le premier sac de collecte ;
une deuxième vanne (132) pour contrôler le flux dans le deuxième sac de collecte ; et
au moins un processeur (812) pour exécuter des instructions lisibles par ordinateur qui, lorsqu'elles sont exécutées par l'au moins un processeur, exécutent les étapes de l'une quelconque des revendications 1 à 11.

13. Appareil selon la revendication 12, dans lequel le premier détecteur est positionné pour détecter la première lumière qui est transmise à travers le flux, et pour détecter la deuxième lumière qui est réfléchie par le flux du premier composant.

14. Appareil selon l'une quelconque des revendications 12 ou 13, dans lequel le deuxième détecteur est positionné pour détecter la deuxième lumière qui est transmise à travers le flux, et pour détecter la première lumière qui est réfléchie par le flux du premier composant.

15. Appareil selon l'une quelconque des revendications 12 à 14, comprenant en outre une pluralité de cellules de séparation et une pluralité de cavités de collecte.
